(19) Europäisches Patentamt
European Patent Office
Office européen des brevets

(11) Publication number: **0 657 455 A1**

(12) # EUROPEAN PATENT APPLICATION

(21) Application number: **94119481.3**

(22) Date of filing: **09.12.94**

(51) Int. Cl.$^{6}$: **C07D 487/08**, A61K 31/44

(30) Priority: **09.12.93 HU 9303506**
**09.12.93 HU 9303507**
**06.10.94 HU 9403506**

(43) Date of publication of application:
**14.06.95 Bulletin 95/24**

(84) Designated Contracting States:
**AT CH DE DK GB LI NL SE**

(71) Applicant: **EGIS GYOGYSZERGYAR**
**Kereszturi ut 30-38**
**H-1106 Budapest (HU)**

(72) Inventor: **Szántay, Dr. Csaba**
**8 Zsombolyai u.**
**H-1113 Budapest (HU)**
Inventor: **Baloch née Kardos, Dr. Zsuzsanna**
**7/b Galgoczy köz**
**H-1125 Budapest (HU)**
Inventor: **Moldvai, Dr. István**
**107 Kossuth L. u.**
**H-1212 Budapest (HU)**
Inventor: **Temesvári née Major, Dr. Eszter**
**52 Rátz L. u.**
**H-1116 Budapest (HU)**
Inventor: **Szántay jr., Dr. Csaba**
**8 Zsombolyai u.**
**H-1113 Budapest (HU)**
Inventor: **Mándi, Dr. Attila**
**12 Riado u.**
**H-1026 Budapest (HU)**
Inventor: **Blasko, Dr. Gábor**
**21 Molnár E. u.**
**H-1113 Budapest (HU)**
Inventor: **Simig, Dr. Gyula**
**25 Hollosy S. u.**
**H-1126 Budapest (HU)**
Inventor: **Lax, Dr. György**
**110 Mogyorodi u.**
**H-1141 Budapest (HU)**
Inventor: **Drabant, Dr. Sándor**
**27 Körömvirág u.**
**H-1171 Budapest (HU)**
Inventor: **Csörgo, Dr. Margit**
**9 Bulyovszky u.**
**H-1174 Budapest (HU)**
Inventor: **Somogyi, Dr. Mária**
**5 Nyugati-tér**
**H-1132 Budapest (HU)**
Inventor: **Lady, Dr. Blanka**
**6 Dagály u.**
**H-1138 Budapest (HU)**
Inventor: **Fekete, Dr. Márton**
**49 Fo u.**
**H-1027 Budapest (HU)**
Inventor: **Szemerédi, Dr. Katalin**
**3 Vörösvári u.**
**H-1035 Budapest (HU)**
Inventor: **Gyertyán, Dr. István**
**33 Koronafürt u.**
**H-1165 Budapest (HU)**
Inventor: **Gigler, Gábor**
**41 Teréz krt.**
**H-1067 Budapest (HU)**
Inventor: **Szállási, Dr. Tamás**
**44 Margit krt.**
**H-1027 Budapest (HU)**

(74) Representative: **Beszédes, Stephan G., Dr.**
**Patentanwalt,**
**Münchener Strasse 80a**
**D-85221 Dachau (DE)**

(54) **Epi-epibatidine derivatives, a process and intermediates for preparing them and epi-epibatidine and medicaments containing the epi-epibatidine derivatives and/or epi-epibatidine and the use of them.**

(57) The subject-matter of the invention are epi-epibatidine derivatives of general formula



Rank Xerox (UK) Business Services
(3.10/3.09/3.3.4)

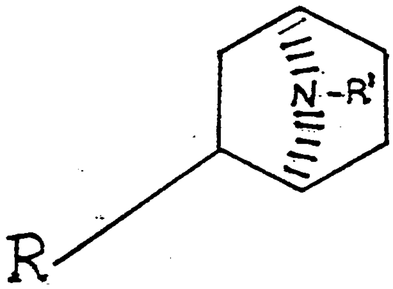

XVII,

wherein

R stands for a $C_{1-4}$-alkyl, $C_{2-4}$-alkenyl, $C_{2-4}$-alkinyl, $C_{3-7}$-cycloalkyl, aryl, heteroaryl, aryl-$C_{1-4}$-alkyl or heteroaryl-$C_{1-4}$-alkyl group, said groups optionally being substituted by 1 or more $C_{1-4}$-alkyl, $C_{2-4}$-alkenyl, $C_{2-4}$-alkinyl, $C_{3-7}$-cycloalkyl, aryl, heteroaryl, aryl-$C_{1-4}$-alkyl, heteroaryl-$C_{1-4}$-alkyl, hydroxy, $C_{1-4}$-alkoxy, phenoxy, halogen, halogeno-$C_{1-4}$-alkyl and/or amino, amido and/or sulfonamido substituent(s), optionally mono- or di-$C_{1-4}$-alkyl-, -$C_{2-4}$-alkenyl- and/or -$C_{2-4}$-alkinyl substituted, and

R' represents hydrogen or a $C_{1-4}$-alkyl, $C_{2-4}$-alkenyl, $C_{2-4}$-alkinyl, $C_{3-7}$-cycloalkyl, $C_{3-7}$-cycloalkenyl, $C_{3-7}$-cycloalkinyl, aryl-$C_{1-4}$-alkyl, aryl, hetero-aryl, halogeno-$C_{1-4}$-alkyl, hydroxy-$C_{1-4}$-alkyl or, preferably $C_{1-4}$-aliphatic; aromatic or heterocyclic, acyl group

with the proviso that,

if R' stands for hydrogen,

R is different from 6-(chloro)-pyrid-3-yl

as well as optically active forms and acid addition salts thereof.

Further aspects of the invention are concerned with a process and intermediates for preparing these compounds as well as medicaments containing them and their use.

The invention is concerned with novel epi-epibatidine derivatives, a process and intermediates for preparing them and epi-epibatidine and medicaments containing the epi-epibatidine derivatives and/or epi-epibatidine and the use of them.

Epi-epibatidine of formula

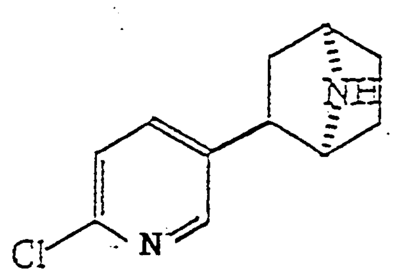

XVIII

has been known from the Prior Art (D. F. Huang, T. Y. Shen: Tetrahedron Letters 34 [1993], pages 4477 to 4480). However, the Prior Art is silent in disclosing any pharmaceutical effect of epi-epibatidine.

Furthermore epibatidine and derivatives of it, in which the 2-position is substituted by a cycloalkyl, aryl, heteroaryl or phenoxy group, optionally substituted, and their analgesic activity have been known (WO 93/18037). However, it contains no reference to epi-epibatidine and its derivatives.

The problem underlying to the invention is to create novel compounds of the epi-epibatidine derivative type having valuable pharmacological effects, particularly analgesic effect, a process and intermediates for preparing them and epi-epibatidine and medicaments containing the novel compounds of the epi-epibatidine derivative type and/or epi-epibatidine and the use of them.

The above surprisingly has been attained by the invention.

The subject-matter of the invention are epi-epibatidine derivatives of general formula

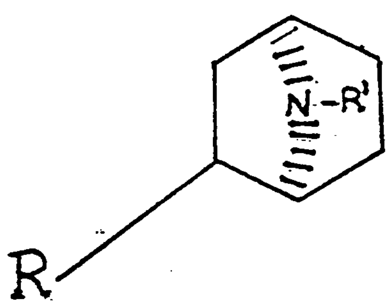

XVII,

wherein

R stands for a $C_{1-4}$-alkyl, $C_{2-4}$-alkenyl, $C_{2-4}$-alkinyl, $C_{3-7}$-cycloalkyl, aryl, heteroaryl, aryl-$C_{1-4}$-alkyl or heteroaryl-$C_{1-4}$-alkyl group, said groups optionally being substituted by 1 or more $C_{1-4}$-alkyl, $C_{2-4}$-alkenyl, $C_{2-4}$-alkinyl, $c_{3-7}$-cycloalkyl, aryl, heteroaryl, aryl-$C_{1-4}$-alkyl, heteroaryl-$C_{1-4}$-alkyl, hydroxy, $C_{1-4}$-alkoxy, phenoxy, halogen, halogeno-$C_{1-4}$-alkyl and/or amino, amido and/or sulfonamido substituent(s), optionally mono- or di-$C_{1-4}$-alkyl-, -$C_{2-4}$-alkenyl- and/or -$C_{2-4}$-alkinyl substituted, and

R' represents hydrogen or a $C_{1-4}$-alkyl, $C_{2-4}$-alkenyl, $c_{2-4}$-alkinyl, $C_{3-7}$-cycloalkyl, $C_{3-7}$-cycloalkenyl, $C_{3-7}$-cycloalkinyl, aryl-$C_{1-4}$-alkyl, aryl, heteroaryl, halogeno-$C_{1-4}$-alkyl, hydroxy-$C_{1-4}$-alkyl or, preferably $C_{1-4}$-aliphatic,aromatic or heterocyclic, acyl group

with the proviso that,

if R' stands for hydrogen,

R is different from 6-(chloro)-pyrid-3-yl

as well as optically active forms and acid addition salts thereof.

The term "$C_{1-4}$-alkyl" relates to straight or branched chained alkyl groups having 1 to 4 carbon atom-(s), e. g. methyl, ethyl, n-propyl or isopropyl, preferably methyl. The term "$C_{2-4}$-alkenyl" relates to straight or branched chained unsaturated aliphatic hydrocarbon groups having at least one double bond and containing 2 to 4 carbon atoms, e. g. vinyl or allyl. The term "$C_{2-4}$-alkinyl" relates to straight or branched chained unsaturated aliphatic hydrocarbon groups having a triple bond and containing 2 to 4 carbon atoms,

e. g. propargyl. The term "$C_{3-7}$-cycloalkyl" relates to cyclic saturated hydrocarbon groups having 3 to 7 carbon atoms, e. g. cyclobutyl, cyclopentyl or cyclohexyl. The term "$C_{3-7}$-cycloalkenyl" relates to cyclic hydrocarbon groups having a double bond and containing 3 to 7 carbon atoms e. g. cyclohexenyl. The term "$C_{3-7}$-cycloalkinyl" relates to cyclic hydrocarbon groups having a triple bond and containing 3 to 7 carbon atoms, e. g. cyclohexinyl. The term "aryl" relates to aromatic groups, e. g. phenyl or naphthyl. The term "aryl-$C_{1-4}$-alkyl" relates to groups in which the aryl and alkyl moiety have the definitions disclosed above, e. g. benzyl or $\beta$-phenylethyl. The term "heteroaryl" relates to mono- or bicyclic heteroaryl groups containing one or more oxygen, sulfur and/or nitrogen atom(s), e. g. pyridyl, pyrimidyl, pyrazinyl, piridazinyl, imidazolyl, thiazolyl, thienyl, furyl, quinonyl or isoquinolyl. The term "$C_{1-4}$-alkoxy" relates to straight or branched chained alkoxy groups having 1 to 4 carbon atom(s), e. g. methoxy, ethoxy, propoxy or isopropoxy, preferably methoxy. The heteroaryl and $C_{1-4}$-alkyl moiety of the "hetero-$C_{1-4}$-alkyl" groups have the definition as disclosed above. The "hydroxy-$C_{1-4}$-alkyl" groups may be straight or branched chained, e. g. hydroxymethyl or 2-(hydroxy)-ethyl. The term "halogen" encompasses the fluorine, chlorine, bromine and iodine atoms. The "halogen-$C_{1-4}$-alkyl" groups may be straight or branched chained, e. g. chloromethyl, 2-(chloro)-ethyl or 3-(iodo)-propyl. The amino group may be optionally substituted by one or two identical or different $C_{1-4}$-alkyl, $C_{2-4}$-alkenyl and/or $C_{2-4}$-alkinyl group(s), e. g. methylamino, ethylamino, dimethylamino or diisopropylamino.

The "acyl" group may be derived from an aliphatic, aromatic or heterocyclic carboxylic acid, e. g. acetyl, propionyl, butyryl, benzoyl or pyridylcarbonyl.

Preferably the $C_{1-4}$-alkyl group(s) which may be represented by R and/or R' and/or by which the group represented by R may be substituted is/are such having 1 or 2, particularly 1, carbon atom(s) and/or the $C_{2-4}$-alkenyl and/or $C_{2-4}$-alkinyl group(s) which may be represented by R and/or R' and/or by which the group represented by R may be substituted is/are such having 2 or 3, particularly 2, carbon atoms and/or the $C_{3-7}$-cycloalkyl group(s) which may be represented by R and/or R' and/or by which the group represented by R may be substituted is/are such having 5 to 7, particularly 5 or 6, above all 6, carbon atoms and/or the $C_{3-7}$-cycloalkenyl or $C_{3-7}$-cycloalkinyl group which may be represented by R' is such having 5 to 7, particularly 5 or 6, above all 6, carbon atoms and/or the alkyl part of the aryl-$C_{1-4}$-alkyl group(s) which may be represented by R and/or R' and/or by which the group represented by R may be substituted is/are such having 1 or 2, particularly 1, carbon atom(s) and/or the alkyl part of the heteroaryl-$C_{1-4}$-alkyl and/or halogeno-$C_{1-4}$-alkyl and/or mono- or di-$C_{1-4}$-alkyl substituted amino-, amido- and/or sulfonamido group(s) by which the group represented by R may be substituted is/are such having 1 or 2, particularly 1, carbon atom(s) and/or the $C_{2-4}$-alkenyl and/or $C_{2-4}$-alkinyl part of the mono- or di-$C_{2-4}$-alkenyl and/or -$C_{2-4}$-alkinyl substituted amino-, amido- and/or sulfonamido group(s) by which the group represented by R may be substituted is/are such having 2 or 3, particularly 2, carbon atom(s) and/or the $C_{1-4}$-alkoxy group(s) by which the group represented by R may be substituted is/are such having 1 or 2, particularly 1, carbon atom(s) and/or the alkyl part of the halogeno-$C_{1-4}$-alkyl and/or hydroxy-$C_{1-4}$-alkyl group which may be represented by R' is such having 1 or 2, particularly 2, carbon atom(s) and/or the $C_{1-4}$-aliphatic acyl group which may be represented by R' is such having 1 or 2, particularly 2, carbon atom(s) and/or the aryl group(s) which may be represented by R and/or R' and/or by which the group represented by R may be substituted is/are [a] phenyl and/or naphthyl group(s), particularly the former one(s), and/or the aryl part of the aryl-$C_{1-4}$-alkyl group(s) which may be represented by R and/or R' and/or by which the group represented by R may be substituted is/are [a] phenyl and/or naphthyl group(s), particularly the former one(s), and/or the heteroaryl group(s) which may be represented by R and/or R' and/or by which the group represented by R may be substituted is/are 5- to 7-membered, particularly 5- or 6-membered, in the heterocyclic ring (part), optionally with a benzene ring condensed to it/them, and/or such having 1 or more nitrogen, oxygen and/or sulfur atom(s) in the heterocylic ring (part), particularly pyridyl, pyrimidyl, pyrazinyl, pyridazinyl, imidazolyl, thiazolyl, thienyl, furyl, quinolyl and/or isochinolyl group(s), and/or the heteroaryl part of the heteroaryl-$C_{1-4}$-alkyl group(s) which may be represented by R and/or by which the group represented by R may be substituted is/are 5- to 7-membered, particularly 5- or 6-membered, in the heterocyclic ring (part), optionally with a benzene ring condensed to it/them, and/or such having 1 or more nitrogen, oxygen and/or sulfur atom(s), particularly the former one(s) in the heterocyclic ring (part), particularly pyridyl, pyrimidyl, pyrazinyl, pyridazinyl, imidazolyl, thiazolyl, thienyl, furyl, quinolyl and/or isochinolyl group(s), and/or the halogen atom(s) by which the group represented by R may be substituted is/are chlorine and/or fluorine, particularly the former one(s), and/or the halogen atom(s) of the halogeno-$C_{1-4}$-alkyl group(s) which may be represented by R' and/or by which the group represented by R may be substituted is/are chlorine and/or fluorine, particularly the former one(s), and/or the aromatic ring of the aromatic acyl group(s), which may be represented by R' is a benzene or naphthalene ring, particularly the former one, and/or the heterocyclic ring of the heterocyclic acyl group(s) which may be

represented by R' is 5- to 7-membered, particularly 5- or 6-membered, in the heterocyclic ring (part), optionally with an benzene ring condensed to it/them, and/or such having 1 or more nitrogen, oxygen and/or sulfur atom(s), particularly the former one(s), in the heterocyclic ring (part), particularly pyridyl, pyrimidyl, pyrazinyl, pyridazinyl, imidazolyl, thiazolyl, thienyl, furyl, quinolyl and/or isochinolyl group.

It is also preferred that R stands for a phenyl or pyridyl group, optionally substituted by halogen or $C_{1-4}$-alkoxy.

Furthermore it is preferred that R stands for a 4-(fluoro)-phenyl, 4-(chloro)-phenyl, 6-(methoxy)-pyrid-3-yl, pyridyl, 1,3-di-(chloro)-phenyl, 1,3-di-(methoxy)-phenyl or 6-(ethoxy)-pyridyl group.

Moreover it is preferred that R' stands for hydrogen or a acetyl group.

A preferred group of the epi-epibatidine derivatives according to the invention are those in which R is a phenyl or pyridyl group, optionally substituted by halogen or $C_{1-4}$-alkoxy, and R' stands for hydrogen or a $C_{1-4}$-alkanoyl group.

A particularly preferred group of epi-epibatidine derivatives according to the invention are those in which R stands for a 4-(fluoro)-phenyl, 4-(chloro)-phenyl, 6-(methoxy)-pyrid-3-yl, pyridyl, 1,3-di-(chloro)-phenyl, 1,3-di-(methoxy)-phenyl or 6-(ethoxy)-pyridyl group and R' represents hydrogen or an acetyl group.

Suitably the acid addition salts of the epi-epibatidine derivatives according to the invention are pharmaceutically acceptable ones. They may have been formed with inorganic acids, e. g. hydrogen halides, such as hydrochloric acid or hydrogen bromide, or sulfuric acid, phosphoric acid or perhalogenic acids, such as perchloric acid, or organic carboxylic acids, e. g. formic acid, acetic acid, propionic acid, glycolic acid, maleic acid, hydroxy-maleic acid, ascorbic acid, citric acid, malic acid, oxalic acid, salicylic acid, lactic acid, cinnamic acid, benzoic acid, phenyl-acetic acid, p-amino-benzoic acid, p-hydroxy-benzoic acid or p-amino-salicylic acid, or alkyl sulfonic acids, e. g. methanesulfonic acid or ethanesulfonic acid, cycloaliphatic sulfonic acids, e. g. cyclohexylsulfonic acid, or arylsulfonic acids, e. g. p--toluene-sulfonic acid, p-bromo-phenyl-sulfonic acid, naphthylsulfonic acid or sulfanilic acid, or amino acids, e. g. aspartic acid, N-(acetyl)-aspartic acid or N-(acetyl)-glutaminicacid.

The epi-epibatidine derivatives of the general formula XVII according to the invention are chiral and may be racemates or optically active isomers. The invention encompasses the racemic and optically active forms of the epi-epibatidine derivatives of general formula XVII according to the invention.

The subject-matter of the invention is also a process for preparing the epi-epibatidine derivatives according to the invention and epi-epibatidine of formula XVII in which R stands for a 6-(chloro)-pyrid-3-yl group and R' represents hydrogen

(formula

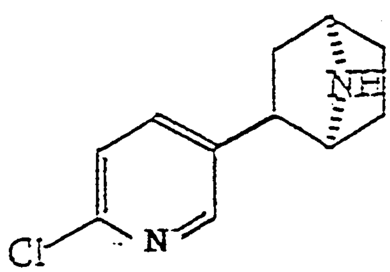

XVIII),

which is characterized by

a) cyclising a racemic or optically active substituted aminocyclohexane of general formula

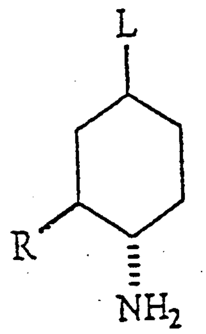

I,

wherein

L represents a leaving group and

R is as above defined without exclusion of the 6-(chloro)-pyrid-3-yl group for the case of stopping after cyclisation to epi-epibatidine or

b) reducing a racemic or optically active substituted nitrocyclohexane of general formula

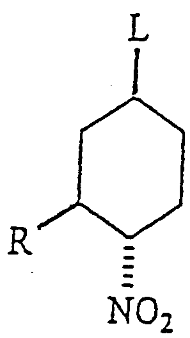

II,

wherein

L represents a leaving group and

R is as above defined without exclusion of the 6-(chloro)-pyrid-3-yl group for the case of stopping after cyclisation to epi-epibatidine,

and cyclising the substituted nitrocyclohexane of general formula I thus obtained

and in a manner known per se, if desired, alkylating, alkenylating, alkinylating, cycloalkylating, cycloalkenylating, cycloalkinylating, arylating, heteroarylating or acylating the epi-epibatidine (derivative) of general formula XVII, wherein R' stands for hydrogen, thus obtained and/or, if desired, converting the epi-epibatidine (derivative) of general formula XVII, thus obtained into an acid addition salt thereof and/or, if desired, liberating from the acid addition salt of the epi-epibatidine (derivative) of general formula XVII thus obtained the free epi-epibatidine (derivative) of general formula XVII and/or converting the latter into another acid addition salt thereof and/or, if desired, resolving the racemic epi-epibatidine (derivative) of general formula XVII or an acid addition salt thereof thus obtained into the optically active isomers thereof and/or racemising the optically active isomers.

In the process according to the invention as starting material adventageously a substituted aminocyclohexane or nitrocyclohexane of general formula I or II, respectively, wherein L stands for a $C_{1-4}$-alkylsulfonyloxy or arylsulfonyloxy group optionally substituted by [a] $C_{1-4}$-alkyl group(s) or halogen(s), particularly a methanesulfonyloxy, p-toluenesulfonyloxy or p-bromo-phenylsulfonyloxy group, is used. Most particularly preferred is to use as starting material a substituted aminocyclohexane or nitrocyclohexane of general formula I or II, respectively, wherein L stands for a methanesulfonyloxy group.

The cyclisation of variant a) and of the second step of variant b) of the process according to the invention is advantageously carried out under heating, i. e. at elevated temperature, particularly at the boiling point of the reaction mixture. Advantageously the cyclisation is performed in a, preferably anhydrous, aprotic solvent as medium. For this purpose particularly halogenated hydrocarbons, most particularly methylene chloride, chloroform or chlorobenzene, or aromatic hydrocarbons, most particularly benzene, toluene or xylene, or a mixture thereof are used. The cyclisation may be carried out under an inert atmosphere, e. g. argon. The epi-epibatidine derivative of general formula XVII or epi-epibatidine of formula

XVIII, respectively, thus obtained may be isolated from the reaction mixture in a known manner, e. g. by cooling the reaction mixture to room temperature, shaking the mixture with an agueous alkali hydroxide solution, separating the layers, extracting the agueous phase with a suitable organic solvent, for example, dichloromethane, washing, drying and evaporating the combined organic extracts. The epi-epibatidine derivative of general formula XVII or epi-epibatidine of formula XVIII, respectively, thus obtained may be purified by crystallization or column chromatography, if necessary.

In variant b) of the process according to the invention the reduction of the substituted nitrocyclohexane of general formula II can be performed by catalytic hydrogenation or chemical reduction. Preferably the catalytic hydrogenation is carried out in the presence of a palladium catalyst, e. g. palladium on charcoal. Suitably catalytic hydrogenation may be carried out in a polar solvent, preferably a lower alkanol, e. g. methanol or a mixture of such in the presence of an acid at a temperature of from 0 to 30°C under atmospheric pressure or a slight overpressure. It is preferred to work at room temperature under atmospheric pressure. The substituted aminocyclohexane of general formula I thus obtained can be isolated by removing the catalyst by filtration, evaporating the filtrate, dissolving the residue in a diluted alkaline solution, extracting with a solvent non miscible with water, e. g. a chlorinated hydrocarbon, aromatic hydrocarbon, ether, ethyl acetate or preferably dichloromethane or a mixture of such, washing, drying and evaporating the organic layer. The residue can be purified by crystallization or column chromatography, if necessary.

Advantageously the chemical reduction is carried out by Bechamps reduction or in glacial acetic acid with zinc or in hydrochloric acid with zinc, iron or tin or with stannous(II)-chloride. Suitably the last mentioned reduction may be performed in a polar organic solvent, e. g. lower alkanol or tetrahydrofurane, or a mixture of such. Chemical reduction may also be accomplished in a neutral medium.

The use of stannous(II)-chloride proved to be particularly preferred. This reduction is preferably carried out in a polar solvent, particularly in ethanol, as medium. Conveniently the reduction with stannous(II)-chloride is carried out at an elevated temperature, preferably at the boiling point of the reaction mixture. The substituted aminocyclohexane of general formula I may be isolated by cooling the reaction mixture, adding to the solution at room temperature a solvent non-miscible with water, e. g. a chlorinated hydrocarbon, preferably chloroform, or a mixture of such and making the solution slightly alkaline. The precipitated product is removed by filtration, washed with a solvent non-miscible with water and the combined organic layers are washed, dried and evaporated. The substituted aminocyclohexane of general formula I thus obtained may be purified by crystallization or column chromatography, if necessary.

An epi-epibatidine derivative of general formula XVII, wherein R' stands for hydrogen, or epi-epibatidine of formula XVIII thus obtained may be alkylated, alkenylated, alkinylated, cycloalkylated, cycloalkenylated, cycloalkinylated, arylated, heteroarylated or acylated.

The optional acylation may be carried out with the aid of organic carboxylic acids or sulfonic acids containing the acyl group to be introduced or with functional derivatives thereof, e. g. acid halides, acid anhydrides or esters. As acid halide preferably acid chlorides may be used.

The optional acylation with a carboxylic acid or sulfonic acid is preferably carried out in an aprotic dipolar solvent, e. g. dimethyl formamide, dimethyl sulfoxide or acetonitrile, or a mixture of such in the presence of a condensing agent. For this purpose preferably condensing agents of the carbodiimide type are used, e. g. dicyclohexylcarbodiimide or carboxy-diimidazole. Acylation is preferably carried out at a temperature of from 0 to 40°C.

The optional acylation with an acid anhydride is preferably carried out in an organic solvent, particularly an apolar aprotic solvent, e. g. halogenated aliphatic hydrocarbon or aromatic hydrocarbon, such as benzene, chloroform or dichloromethane, or a mixture of such. The reaction temperature may vary within wide ranges, one preferably works at room temperature. Suitably the reaction is carried out in the presence of an acid binding agent. For this purpose inorganic bases, e. g. alkali carbonates, such as sodium carbonate, or organic bases, e. g. pyridine, may be used. Certain organic bases, e. g. pyridine, may play the role of both the solvent and the acid binding agent.

Suitably the optional acylation with an acid halide is carried out in an inert organic solvent. As reaction medium advantageously an aliphatic ether, e. g. diethyl ether, cyclic ether, e. g. tetrahydrofurane, an aliphatic hydrocarbon, optionally halogenated, e. g. chloroform, or an aromatic hydrocarbon, e. g. benzene, or a mixture of such is used. The reaction temperature may vary within wide ranges, one preferably works at ambient temperature. Suitably the reaction is carried out in the presence of an acid binding agent. For this purpose preferably inorganic bases, e. g. alkali carbonates, such as sodium carbonate, or tertiary organic bases, e. g. triethyl amine, N-methyl morpholine or pyridine, are used. Certain organic bases, e. g. pyridine, may play the role of both the solvent and the acid binding agent.

The optional alkylation of the epi-epibatidine derivatives of general formula XVII, wherein R' stands for hydrogen, and of epi-epibatidine of formula XVIII may be carried out by methods known per se. As alkylating agent e. g. dimethyl sulfate, methyl iodide or ethyl iodide may be used. Said alkylation reaction is carried out preferably in the presence of a base, e. g. an alkali hydroxide, carbonate or bicarbonate.

The optional conversion of the epi-epibatidine derivatives of general formula XVII and epi-epibatidine of formula XVIII into their acid addition salts may be carried out in a known manner in an inert organic solvent, e. g. lower aliphatic alkanol, acetone, ethyl acetate, ether, acetonitrile, dioxane or tetrahydrofurane or a mixture of such. One may proceed by dissolving the epi-epibatidine derivative of general formula XVII or epi-epibatidine of formula XVIII in one of the above solvents and acidifying the solution by adding the corresponding acid or a solution thereof formed with one of the above solvents. The precipitated acid addition salt may be isolated by known methods, e. g. filtration.

The optional resolution of a racemic epi-epibatidine derivative of general formula XVII or of racemic epi-epibatidine of formula XVIII, respectively, and thus separation into the optically active isomers may be carried out by methods known per se. Thus one may proceed by reacting a racemic epi-epibatidine derivative of general formula XVII or racemic epi-epibatidine of formula XVIII, respectively, with an optically active acid, e. g. optically active tartaric acid, di-0,0'-o-toluyl-tartaric acid or dibenzoyl tartaric acid, separating the diastereomeric salts thus obtained, e. g. by fractionated crystallization and liberating the optically active epi-epibatidine derivative of general formula XVII or optically active epi-epibatidine of formula XVIII from its salt.

The optically active epi-epibatidine derivatives of general formula XVII or optically active epi-epibatidine of formula XVIII, respectively, may also be prepared by subjecting a chiral intermediate product of the synthesis of the starting compound to resolution and carrying out with the optically active intermediate compound thus obtained the further steps of the synthesis leading to the desired end-product of the general formula XVII or formula XVIII, respectively.

One may proceed preferably by subjecting a racemic substituted 1-(nitro)-cyclohexane-4-ol of general formula

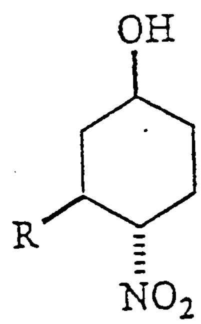

III,

wherein

R is as above defined without exclusion of the 6-(chloro)-pyrid-3-yl group for the case of stopping after cyclisation to -epi-epibatidine,

to resolution and using the optically active substituted 1-(nitro)-cyclohexane-4-ol of formula III thus obtained for the further steps of the synthesis. The racemic substituted 1-(nitro)-cyclohexane-4-ol of general formula III preferably is separated into the optically active isomers by acylating with an optically active menthyl chloroformiate, separating the diastereomeric salts thus formed by crystallization and removing the menthyl group to yield the desired optically active substituted 1-(nitro)-cyclohexane-4-ol of general formula III.

The starting materials of the general formula II may have been prepared as follows:

The particular advantage of the following synthesis is that as starting materials commercially readily available nitromethane of formula

$H_3C - NO_2$ XII

and methylvinylketone of formula

$$H_2C = \overset{H}{\overset{|}{C}} - \overset{O}{\overset{||}{C}} - CH_3 \qquad XI$$

are used. These compounds are reacted to yield the 1-(nitro)-pentane-4-one of formula

$$NO_2 - (CH_2)_3 - \overset{O}{\overset{||}{C}} - CH_3 \qquad X.$$

The reaction may be carried out as described by D. E. Bergbreiter and J. J. Lalonde (J. Org. Chem. 52 - [1987], 1 601 to 1 603).

In the next step of the synthesis the 1-(nitro)-pentane-4-one of formula X is brominated. Preferably the reaction is carried out with elementary bromine in a lower alkanol, advantageously methanol. Conveniently the bromination is performed at ambient temperature and care is taken that the reaction temperature should not be higher than 40 °C. The acetale type ether bond formed in the course of the reaction is hydrolysed. The 1-(bromo)-5-(nitro)-pentane-2-one of formula

$$NO_2 - (CH_2)_3 - \overset{O}{\overset{||}{C}} - CH_2 - Br \qquad IX$$

thus obtained may be isolated by extracting the aqueous solution with a solvent non-miscible with water, e. g. chlorinated hydrocarbon, aromatic hydrocarbon, ethyl acetate or preferably ether, or a mixture of such. Suitably the extract is first washed acid-free with an alkali solution and then neutral with water, dried and evaporated. The 1-(bromo)-5-(nitro)-pentane-2-one of formula IX may be purified by column chromatography, if desired.

The 1-(bromo)-5-(nitro)-pentane-2-one of formula IX is reacted with a triaryl phosphine to yield a phosphonium salt [5-(nitro)-pentane-2-one]-triaryl-phosphoniumbromide of general formula

$$NO_2 - (CH_2)_3 - \overset{O}{\overset{||}{C}} - CH_2 - \underset{Br^-}{P^+(Ar)_3} \qquad VIII,$$

wherein

Ar stands for an aryl group, preferably phenyl group.

The reaction is advantageously carried out by using triphenyl phosphine. It is advantageously performed in an apolar aprotic solvent, preferably an aromatic hydrocarbon, particularly benzene, or a mixture of such. One may proceed preferably by adding to the solution of the 1-(bromo)-5-(nitro)-pentane-2-one of formula IX in an apolar aprotic solvent a solution of triphenyl phosphine in the same solvent. The reaction may be carried out at 10 to 30 °C, preferably at room temperature. The oily product gradually crystallizes on standing. The crystalline [5-(nitro)-pentane-2-one]-triaryl-phosphonium bromide, preferably [5-(nitro)-pentane-2-one]-triphenylphosphonium bromide, of formula VIII may be isolated by filtration and purified by washing.

The phosphonium salt [5-(nitro)-pentane-2-one]-triaryl-phosphonium bromide, preferably [5-(nitro)-pentane-2-one]-triphenyl-phosphonium bromide, of general formula VIII is then converted into a [5-(nitro)-pentane-2-one]-triaryl-phosphorane, preferably [5-(nitro)-pentane-2-one]-triphenyl-phosphorane, of general formula

$$NO_2 - (CH_2)_3 - \overset{O}{\overset{||}{C}} - CH = P(Ar)_3 \qquad VII,$$

wherein

Ar stands for an aryl group, preferably phenyl group.

This reaction is preferably carried out as follows. The phosphonium salt [5-(nitro)-pentane-2-one]-triaryl-phosphonium bromide, preferably [5-(nitro)-pentane-2-one]-triphenyl-phosphonium bromide, of general formula VIII is dissolved in an aprotic apolar solvent non-miscible with water, e. g. a halogenated aliphatic hydrocarbon, such as dichloromethane, or a mixture of such solvents and admixed with a diluted alkali hydroxide solution, e. g. sodium or potassium hydroxide solution, at ambient temperature under stirring. The layers are separated, the organic phase is washed, dried and evaporated.

The [5-(nitro)-pentane-2-one]-triaryl-phosphorane of general formula VII thus obtained is reacted with an aldehyde of general formula

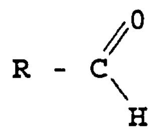 VI,

wherein

R is as above defined without exclusion of the 6-(chloro)-pyrid-3-yl group for the case of stopping after cyclisation to epi-epibatidine.

In the preparation of epi-epibatidine of formula XVIII (formula XVII with R = 6-(chloro)-pyrid-3-yl group and R' = hydrogen) 6-(chloro)-pyridine-3-aldehyde [6-(chloro)-nicotine aldehyde] is used as aldehyde of general formula VI. The reaction may be carried out in an anhydrous aprotic solvent, preferably a halogenated aliphatic hydrocarbon, e. g. dichloromethane, or a mixture of such. The reaction may be performed at elevated temperature, preferably at the boiling point of the reaction mixture. One may proceed preferably by adding to a solution of a [5-(nitro)-pentane-2-one]-triaryl-phosphorane, preferably [5-(nitro)-pentane-2-one]-triphenyl-phosphorane, of general formula VII in an anhydrous aprotic solvent a solution of the aldehyde of general formula VI in the same solvent. The reaction mixture is preferably worked up by cooling, washing, drying and evaporation. The olefine substituted 3-(oxo)-6-(nitro)-hexa-1-ene of general formula

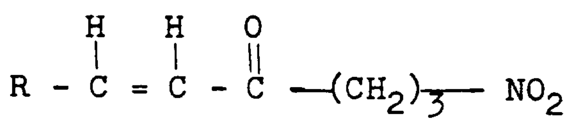 V,

wherein

R is as above defined without exclusion of the 6-(chloro)-pyrid-3-yl group for the case of stopping after cyclisation to epi-epibatidine,

may be purified by crystallization or column chromatography.

The 6-(chloro)-nicotine aldehyde of general formula VI used in this synthesis for preparing epi-epibatidine of formula XVIII can be prepared from the commercially readily available 6-(chloro)-nicotinic acid by a method described in prior Art (F. E. Ziegler, J. G. Sweeny: Tetrahedron Letters, 1969, 1097 to 1110).

The olefine substituted 3-(oxo)-6-(nitro)-hexa-1-ene of general formula V thus obtained is subjected to cyclisation. Ring closure may be carried out in an anhydrous aprotic organic solvent. As reaction medium preferably cyclic ethers, e. g. tetrahydrofurane, or a mixture of such may be used. The reaction is preferably performed in the presence of a base, particularly potassium fluoride applied on a basic aluminum oxide carrier (D. E. Bergbreiter, J. J. Lalonde: J. Org. Chem. 52 [1987], pages 1601 to 1603). Cyclisation may be carried out at ambient temperature. The nitroketone substituted 1-(nitro)-cyclohexane-4-one of general formula

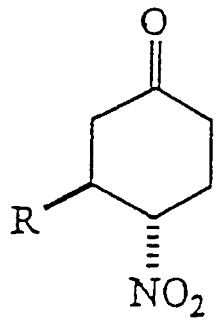

IV,

wherein

R is as above defined without exclusion of the 6-(chloro)-pyrid-3-yl group for the case of stopping after cyclisation to epi-epibatidine,

thus obtained may be purified by crystallization or column chromatography, if desired.

The nitroketone substituted 1-(nitro)-cyclohexane-4-one of general formula IV thus obtained is reduced to a substituted 1-(nitro)-cyclohexane-4-ol of general formula III. The reduction may be carried out with the aid of a complex metal hydride, preferably sodium borohydride, or lithium, sodium and potassium tri-2-butyl borohydride [L-selectride®]. One proceeds preferably by using sodium borohydride. The reduction with sodium borohydride is preferably performed in a lower alkanol, advantageously ethanol, under cooling, preferably at a temperature of about 0 ° C. The reduction having been completed the excess of the reducing agent is decomposed by adding a solvent containing an oxo group, e. g. acetone, whereupon the solvent is removed and the hydroxy compound substituted 1-(nitro)-cyclohexane-4-ol of general formula III is preferably isolated as follows: the evaporation residue is dissolved in a solvent non-miscible with water, e. g. a halogenated hydrocarbon, particularly chloroform, aromatic hydrocarbon, ether or ethyl acetate, or a mixture of such, washing, drying and evaporating the organic phase. The substituted 1-(nitro)-cyclohexane-4-olof general formula III thus obtained may be purified by crystallization or chromatography, if desired.

In the next step a leaving group L is introduced into the nitroalcohol substituted 1-(nitro)-cyclohexane-4-ol of general formula III to yield a compound (substituted nitrocyclohexane) of general formula II, a starting substance of the process according to the invention. The reaction is preferably carried out by reacting the substituted 1-(nitro)-cyclohexane-4-ol of general formula III with the corresponding sulfonyl halide, advantageously methanesulfonyl chloride. The acylating agent is preferably used in an excess. Advantageously the reaction may be performed in an apolar aprotic solvent, e. g. halogenated hydrocarbon, such as dichloromethane or chloroform, or a mixture of such in the presence of base, e. g. pyridine. One proceeds preferably by carrying out the reaction in a mixture of dichloro methane and pyridine. The reaction is carried out preferably at ambient temperature. The substituted nitrocyclohexane of general formula II is isolated preferably as follows: the reaction mixture is evaporated, the residue is dissolved in an organic solvent non-miscible with water, e. g. a halogenated hydrocarbon, particularly chloroform, aromatic hydrocarbon, ether or ethyl acetate, or a mixture of such, extracting the organic solution with an inorganic base, e. g. alkali carbonate, extracting the aqueous layer with the same organic solvent non-miscible with water mentioned above, combining the organic layers, drying and evaporating the same. The substituted nitrocyclohexane of general formula II thus obtained may be purified by crystallization or column chromatography, if desired.

A subject-matter of the invention are also substituted aminocyclohexanes of general formula

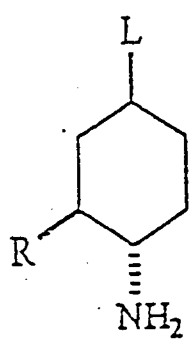

I,

wherein

L represents a leaving group and

R is as above defined there being no exclusion of the 6-(chloro)-pyrid-3-yl group in view of epi-epibatidine,

substituted nitrocyclohexanes of general formula

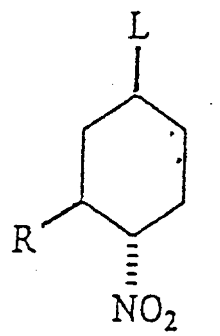

II,

wherein

L represents a leaving group and

R is as above defined there being no exclusion of the 6-(chloro)-pyrid-3-yl group in view of epi-epibatidine,

substituted 1-(nitro)-cyclohexane-4-ols of general formula

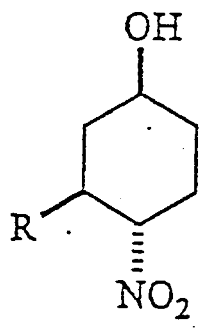

III,

wherein

R is as above defined there being no exclusion of the 6-(chloro)-pyrid-3-yl group in view of epi-epibatidine,

substituted 1-(nitro)-cyclohexane-4-ones of general formula

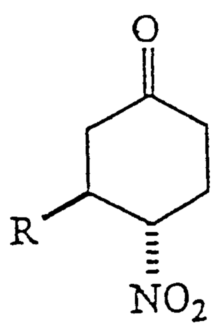

IV,

wherein

R is as above defined there being no exclusion of the 6-(chloro)-pyrid-3-yl group in view of epi-epibatidine,

and substituted 3-(oxo)-6-(nitro)-hexa-1-enes of general formula

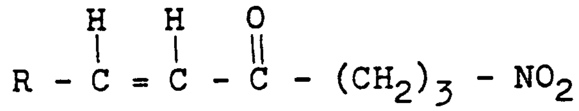

V,

wherein

R is as above defined there being no exclusion of the 6-(chloro)-pyrid-3-yl group in view of epi-epibatidine.

For these apply the same preferences of the groups as for the epi-epibatidine derivatives of general formula XVII.

The above compounds are the cause of the outstanding pharmacological effects of the final products epi-epibatidine (derivatives) of general formula XVII or XVIII, respectively.

A subject-matter of the invention are also medicaments which are characterized in that they contain 1 or more epi-epibatidine derivative(s) according to the invention and/or epi-epibatidine and/or acid addition salt-(s) thereof as [an] active ingredient(s), advantageously in admixture with 1 or more inert solid and/or liquid pharmaceutical carrier(s) and/or auxiliary agent(s).

Surprisingly it has been found that the epi-epibatidine derivatives of general formula XVII and the epi-epibatidine of formula XVIII show outstanding pharmacological, particularly analgesic activity. They are highly potent analgesics and the activity thereof is superior to that of morphine. The epi-epibatidine of formula XVIII exhibits particularly high acitity. Own results suggest that the analgesia induced by these compounds is not mediated through opioid receptor agonism.

The activity of the epi-epibatidine derivatives of general formula XVII and of epi-epibatidine of formula XVIII is shown by the following tests:

The toxicity of the epi-epibatidine derivatives of general formula XVII and of epi-epibatidine of formula XVIII is very low. Thus the epi-epibatidine of formula XVIII does not destroy mice in a subcutaneous dose of 5 mg/kg within 24 hours.

Hot plate test in mice Method

A modification of method of Eddy et al. (1953) Eddy, N. B., Leimback D.: Synthetic analgesics II. Dithienylbutenyl and dithienylbutylamines. J. Pharmacol. Exp. Ther. 107 /1953/, 385 to 393 was used. NMRI male mice (20 to 25 g body weight) were dropped on a hot plate (56.0 ± 0.5 °C) and the latency time elapsed until licking the forepaws was measured. The compounds were administered subcutaneously and 15, 30, 45 and 60 minutes later the reaction time was measured again. Animals were regarded as positive if they produced at least twice 2.5-fold reaction time extension as related to their control values. The results are summarized in the following table 1.

Table 1

| Test Compound Example No. | Designation | $ED_{50}$ mg/kg s.c. |
|---|---|---|
| 6 | Epi-Epibatidine | 0.7 |
| 33 | (±)-2β-[Pyrid-3'-yl]-7α-azabicyclo[2.2.1]heptane | ∼ 10 |
| 26 | (±)-2β-[6'-(Methoxy)-pyrid-3'-yl]-7α-azabicyclo[2.2.1]heptane | 3.1 |
| 12 | (±)-2β-[1'-(Fluoro)-phen-4'-yl]-7α-azabicyclo[2.2.1]heptane | 2.1 |
| 19 | (±)-2β-[1'-(Chloro)-phen-4'-yl]-7α-azabicyclo[2.2.1]heptane | 6.3 |
| 42 | (±)-2β-[1',3'-Di-(chloro)-phen-4'-yl]-7α-azabicyclo[2.2.1]heptane | 1.8 |
| 48 | (±)-2β-[1',3'-Di-(methoxy)-phen-4'-yl]-7α-azabicyclo[2.2.1]heptane | 2.9 |
| 49 | (±)-2β-[6'-(Ethoxy)-pyrid-3'-yl]-7α-azabicyclo[2.2.1]heptane | 3.5 |
| Reference substance A | Morphine-HCl | 2.7 |
| Reference substance B | Codeine-HCl | 9.4 |

According to these results the epi-epibatidine derivatives according to the invention were more potent than codeine, their analgesic activities were as potent as that of morphine. The analgesic potency of epi-epibatidine (Example 6) was 4 times stronger than that of morphine and 13 times stronger than that of codeine in this model.

Acetic acid induced writhing test in mice Method

Groups of 12 mice weighing 20 to 25 g were treated with 0.75% (v/v) acetic acid in a volume of 20 ml/kg administered intraperitoneally according to the method of Koster, R., Anderson, M., de Beer, E. J.: Acetic acid for analgesic screening, Fed. Proc. 18 [1959], 412. Between 5 and 10 minutes after the administration of acetic acid the total number of the typical "writhing" reactions was counted and expressed as percent inhibition compared to the concurrent controls. The test compounds were applied subcutaneously partly with a pretreatment period of 30 min. and partly without such. Statistical evaluation was performed by Student's t test and $ID_{50}$ values were determined by plotting the log-dose versus percentage response as compared to vehicle-treated animals. 10 mg/kg s.c. 17-(allyl)-4,5α-(epoxy)-3,14-di-(hydroxy)-morphinan-6-one {Naloxone} was administered 15 minutes before drug treatment. The results are shown in the following table 2.

Table 2

| Test Compound |  | $ID_{50}$ mg/kg s.c. |  |
|---|---|---|---|
| Example No. | Designation | Without pretreatment | Pretreatment with 10 mg/kg 17-(allyl)-4,5α-(epoxy)-3,14-di-(hydroxy)-morphinan-6-on {naloxone} s.c. |
| 6 | Epi-epibatidine | 2.6 | 3.2 |
| Reference substance A | Morphine-HCl | 0.6 | 46.8 |
| Reference substance B | Codeine-HCl | 9.9 | 45.4 |

In the mice pretreated with the opioid antagonist 17-(allyl)-4,5α-(epoxy)-3,14-di-(hydroxy)-morphinan-6-one {Naloxone} (10 mg/kg s.c.) for 15 minutes the $ID_{50}$ values of morphine (reference substance A) and codeine (reference substance B) in antinociception increased by about 80 and 5 fold, respectively. On the other hand, 17-(allyl)-4,5α-(epoxy)-3,14-di-(hydroxy)-morphinan-6-one {Naloxone} was inactive in antagonizing the antinociception of the test compound epi-epibatidine {Example 6} antinociception. These results

suggest that the analgesia induced by the epi-epibatidine derivatives according to the invention and epi-epibatidine is not mediated through opioid receptor agonism.

Avantageously the medicaments according to the invention are in the form of pharmaceutical preparations (compositions). These may be prepared by methods of the pharmaceutical technique known per se.

The pharmaceutical preparations according to the invention may be for oral or parenteral administration. The oral compositions are preferably tablets, capsules, dragées or pills. The parenteral compositions are preferably injectable solutions. The pharmaceutical preparations of the present invention contain 1 or more conventional pharmaceutical carrier(s) and/or auxiliary agent(s). The oral preparations may contain e.g. calcium carbonate, magnesium stearate, talc and/or starch as carrier(s). The injectable solutions may contain water as solvent but an isotonic sodium chloride solution may also be used. The pharmaceutical preparations may contain as conventional auxiliary agent(s) e.g. [a] wetting, emulsifying, suspending and/or stabilizing agent(s) and/or salt(s) to modify the osmotic pressure and/or buffer(s).

The daily dosage of the epi-epibatidine derivatives of general Formula XVII and of epi-epibatidine of formula XVIII may vary within wide ranges and is always determined by the physician taking into consideration the circumstances of the given treatment on a case-by-case basis. The daily oral dose is generally from about 0.5 mg to about 50 mg. The daily parenteral dosage is generally from about 0.1 mg to about 10 mg. The above values relate to a body weight of 70 kg.

The epi-epibatidine derivatives of general Formula XVII and epi-epibatidine of formula XVIII exhibit a significant effect on the central nervous system, too.

A subject-matter of the invention is also the use of the epi-epibatidine derivatives according to the invention and/or of epi-epibatidine and/or of acid addition salts thereof for the preparation of medicaments, particularly with analgesic activity.

The treatment of mammals including humans comprises administering to an individual in need of such treatment an analgesically active amount of a epi-epibatidine derivative of general Formula XVII and/or of epi-epibatidine of formula XVIII and/or of a pharmaceutically acceptable acid addition salt thereof.

The invention is illustrated in detail with the aid of the following Examples. In these the ratios of the components of the mixtures for chromatography are always by volume. Moreover in the following Examples unless other indications have been made column chromatography is to be meant as having been carried out on silica gel with the designation "Kieselgel 60" (0.063 to 0.200 mm). Furthermore "brine" is a saturated sodium chloride solution. For the potassium fluoride precipitated on aluminum oxide always the total amount of these 2 substances and the potassium fluoride contents thereof have been indicated.

Example 1

1-[6'-(Chloro)-pyrid-3'-yl]-3-[oxo]-6-[nitro]-hexa-1-ene

To a solution of the 13.5 g (0.0344 mole) of the [5-(nitro)-pentane-2-one]-triphenyl-phosphorane prepared as indicated below in 70 ml of anhydrous dichloromethane a solution of 3.1 g (0.022 mole) of 6-(chloro)-pyridine-3-aldehyde in 70 ml of anhydrous dichloromethane is added. The reaction mixture is heated to boiling for 8 hours in argon atmosphere. The reaction mixture is cooled, the dichloromethane solution is washed subsequently three times with 150 ml of water each and 150 ml of a saturated sodium chloride solution, dried over calcium chloride and evaporated. The dry residue is subjected to chromatography on a silica column and eluted with a 1:1 mixture of n-hexane and ethyl acetate. Thus 4.7 g of the pure desired compound are obtained, yield 84%. Mp.: 97-100 °C. $R_f$ = 0.52.

$IR_{(KBr)}$: 1700, 1680, 1620, 1580, 1550, 1100 $cm^{-1}$.

The [5-(nitro)-pentane-2-one]-triphenyl-phosphorane used as starting substance has been prepared as follows.

a)

1-(Bromo)-5-(nitro)-pentane-2-one

80.0 g (0.61 mole) of 1-(nitro)-pentane-4-one are dissolved in 250 ml of anhydrous methanol, whereupon 31.5 ml (0.61 mole) of bromine are quickly added under cooling with ice. The reaction mixture is stirred for a further 2 hours at a rate that the internal temperature should not exceed 40 °C. To the reaction mixture 250 ml of water are added, the reaction mixture is stirred at room temperature overnight. Next morning the solution is extracted three times with 300 ml of ether each, the ethereal solution is washed with a 10% by weight aqueous sodium carbonate solution free of acid, whereupon it is washed three times with 200 ml of water each and 200 ml of a saturated sodium chloride solution, dried over calcium chloride and

evaporated. The dry residue is subjected to chromatography on a silica column and eluted with a 3:1 mixture of n-hexane and ethyl acetate. Thus 70.4 g of the desired compound are obtained in the form of a faint yellow liquid, yield 55%. $R_f$ = 0.30.
$IR_{(film)}$: 2950, 1720, 640 $cm^{-1}$.

b)
[5-(Nitro)-pentane-2-one]-triphenyl-phosphonium bromide

10.25 g (0.048 mole) of the bromine compound 1-(bromo)-5-(nitro)-pentane-2-one prepared according to section a) are dissolved in 30 ml of anhydrous benzene whereupon a solution of 14.09 g (0.0537 mole) of triphenyl phosphine in 50 ml of anhydrous benzene is added dropwise. The reaction mixture is stirred at room temperature for 48 hours whereby the oily precipitate becomes crystalline. The precipitated salt is filtered and washed with n-hexane. Thus 20.5 g of the desired compound are obtained,
yield 89%, mp.: 70-72 °C.

c)
[5-(Nitro)-pentane-2-one]-triphenyl-phosphorane

8.1 g (0.0171 mole) of the phosphonium salt [5-(nitro)-pentane-2-one]-triphenyl-phosphonium bromide prepared according to section b) are dissolved in 160 ml of dichloromethane and the solution formed is stirred with 136 ml (0,0542 mole) of a 1% by weight aqueous sodium hydroxide solution for 30 minutes. The two phases are separated, the dichloromethane layer is washed three times with 100 ml of water each and with 100 ml of a saturated sodium chloride solution, dried over calcium chloride and evaporated. The dry residue is thoroughly triturated with n-hexane. Thus 4.8 g of the desired compound are obtained, yield 72%, mp.: 94-97 °C.

Example 2

(±)-1α-[Nitro]-2β-[6'-(chloro)-pyrid-3'-yl]-cyclohexane-4-one

1.6 g (0,0063 mole) of 1-[6'-(chloro)-pyrid-3'-yl]-3-[oxo]-6-[nitro]-hexa-1-ene prepared according to Example 1 are dissolved in 50 ml of anhydrous tetrahydrofurane whereupon 4.0 g (0.089 mole) of potassium-fluoride precipitated on aluminum oxide containing 0.0117 mole of potassium fluoride are added. The reaction mixture is stirred at room temperature overnight. The solid product is filtered, washed with 300 ml of ethyl acetate. The combined filtrates are dried over calcium chloride and evaporated. The residue is purified by chromatography on a silica column and elution with a 1:1 mixture of n-hexane and ethyl acetate. Thus 1.1 g of the pure desired product are obtained, yield 59%.
Mp.: 118-121 °C.
$R_f$ = 0.38. $IR_{(KBr)}$: 1710, 1585, 1550, 1100 $cm^{-1}$.

Example 3

(±)-1α-[Nitro]-2β-[6'-(chloro)-pyrid-3'-yl]-cyclohexane-4β-ol

2.8 g (0.0110 mole) of (±)-1α-[nitro]-2β-[6'-(chloro)-pyrid-3'-yl]-cyclohexane-4-one prepared according to Example 2 are dissolved in 200 ml of anhydrous ethanol whereupon 1.2 g (0.0317 mole) of sodium borohydride are added within a period of about one hour and a half in small portions. The excess of the reducing agent is decomposed by careful addition of acetone, the reaction mixture is evaporated in vacuo, the solid residue is dissolved in a mixture of 50 ml of water and 200 ml of chloroform, the mixture is thoroughly shaken and the layers are separated. The aqueous phase is extracted three times with 200 ml of chloroform each. The combined organic layers are washed twice with 200 ml of water each and 100 ml of a saturated sodium chloride solution, dried over calcium chloride and evaporated. Thus 1.9 g of the desired compound are obtained, yield 67%, mp.: 149-153 °C.
$IR_{(film)}$: 3380, 1580, 1570, 1550, 1100, 1080 $cm^{-1}$.
$R_f$ = 0.42 (10 : 1 mixture of chloroform and methanol).

Example 4

(±)-1α-[Nitro]-2β-[6'-(chloro)-pyrid-3'-yl]-4β-[methanesulfonyloxy]-cyclohexane

1.0 g (0.003896 mole) of (±)-1α-[nitro]-2β-[6'-(chloro)-pyrid-3'-yl]-cyclohexane-4β-ol prepared according to Example 3 are dissolved in a mixture of 15 ml of anhydrous dichloromethane and 30 ml of pyridine, whereupon 0.75 ml (0.0097 mole) of methanesulfonyl chloride is added dropwise under cooling with icecold water. The reaction mixture is stirred at room temperature overnight and thereafter the solvent is removed in vacuo. The dry residue is dissolved in a mixture of 50 ml of chloroform and 25 ml of a 10% by weight sodium carbonate solution, the mixture is thoroughly shaken, the phases are separated. The aqueous phase is extracted three times with 50 ml of chloroform each. The combined organic layers are washed three times with 100 ml of water each and 100 ml of a saturated sodium chloride solution, dried over calcium chloride and evaporated. The dry residue is subjected to chromatography on a silica column and eluted with a 1:1 mixture of n-hexane and ethyl acetate. Thus 1.18 g of the desired compound are obtained, yield 91%, mp.: 120-122 °C.
$R_f$ = 0.46
$IR_{(KBr)}$: 1590, 1570, 1540, 1530, 1450, 1350, 1180, 1090 $cm^{-1}$.

Example 5

(±)-1α-[Amino]-2β-[6'-(chloro)-pyrid-3'-yl]-4β-/methanesulfonyloxy/-cyclohexane

1.5 g (0.0048 mole) of (±)-1α-[nitro]-2β-[6'-(chloro)-pyrid-3'-yl]-4β-[methanesulfonyloxy]-cyclohexane prepared according to Example 4 are dissolved in 150 ml of ethanol whereupon 10.76 g (0.0477 mole) of stannous(II)-chloride dihydrate are added. The reaction mixture is heated to boiling for 24 hours, whereupon it is cooled, 200 ml of chloroform are added and the pH is adjusted to 9 by adding a concentrated ammonium hydroxide solution. The precipitated product is filtered, washed with 400 ml of chloroform, the organic phase is washed twice with 200 ml of water and once with 200 ml of a saturated sodium chloride solution, dried over magnesium sulfate and evaporated. Thus 1.1 g of the desired compound are obtained in the form of a colourless oil, yield 80%.
$R_f$ = 0.69 (chloroform : methanol = 10 : 1)

Example 6

Epi-epibatidine

1.1 g (0.0036 mole) of (±)-1α-[amino]-2β-[6'-(chloro)-pyrid-3'-yl]-4β-[methanesulfonyloxy]-cyclohexane prepared according to Example 5 are dissolved in 150 ml of anhydrous toluene and the reaction mixture is heated to boiling under argon overnight. The reaction mixture is then cooled, 25 ml of a 5% by weight sodium hydroxyde solution are added, the phases are thoroughly shaken, the layers are separated. The aqueous phase is extracted ten times with 20 ml of dichloromethane each. The combined organic layers are washed twice with 100 ml of water each and 100 ml of a saturated sodium chloride solution, dried over magnesium sulfate and evaporated. The residue is subjected to chromatography on a silica column and eluted with a 1:1 mixture of chloroform and methanol. Thus 350 mg of the desired compound are obtained, yield 46%, faint yellow oil. $R_f$ = 0.35 (1 : 1 mixture of chloroform and methanol).
$IR_{(film)}$: 3260, 3220, 1580, 1560, 1760, 1200, 1100 $cm^{-1}$.

Example 7

1-[1'-(Fluoro)-phen-4'-yl]-3-[oxo]-6-[nitro]-hexa-1-ene

3.72 g (29.9 millimoles) of 4-(fluor)-benzaldehyde are dissolved in 100 ml of anhydrous dichloromethane. To the solution 15.24 g (38.9 millimoles) of [5-(nitro)-pentane-2-one]-triphenyl-phosphorane prepared according to the part of Example 1 regarding the preparation of its starting substances are added and the reaction mixture is stirred at 70 °C (bath temperature) for 24 hours. The reaction having been completed the dichloromethane solution is washed with 150 ml of water and 150 ml of saturated sodium chloride solution, dried over sodium sulfate and evaporated. The crude product is purified by chromatography and elution with a 3:1 mixture of n-hexane and ethyl acetate. Thus 6.1 g of the desired compound are

obtained in the form of a thick oil, yield 86%.
$R_f$ = 0.3 (3 : 2 mixture of n-hexane and ethylacetate).
$IR_{(film)}$: 1700, 1670, 1620, 1600, 1550, 1500, 1230, 1160, 840, 820 $cm^{-1}$.

Example 8

(±)-1α-[Nitro]-2β-[1'-(fluoro)-phen-4'-yl]-cyclohexane-4-one

0.62 g (2.62 millimoles) of 1-[1'-(fluoro)-phen-4'-yl]-3-[oxo]-6-[nitro]-hexa-1-ene prepared according to Example 7 is dissolved in 20 ml of anhydrous tetrahydrofurane whereupon 1.17 g (26 millimoles) of potassium fluoride precipitated on aluminum oxide containing 3.44 mmoles of potassium fluoride are added and the reaction mixture is stirred at room temperature for 24 hours. The solid phase is removed by filtration, the tetrahydrofurane is distilled off and the crude residue is purified by column chromatography and elution with a 3:1 mixture of n-hexane and ethyl acetate. Thus 0.27 g of the crystalline desired compound are obtained, yield 44%, mp.: 105-110 °C. $R_f$ = 0.2 (ethylacetate : n-hexane = = 1 : 3).
$IR_{(KBr)}$: 1700, 1600, 1530, 1500, 1215, 870, 830 $cm^{-1}$.

Example 9

(±)-1α-[Nitro]-2β-[1'-(fluoro)-phen-4'-yl]-cyclohexane-4β-ol

230 mg (0.97 millimoles) of (±)-1α-[nitro]-2β-[1'-(fluoro)-phen-4'-yl]-cyclohexane-4-one prepared according to Example 8 are dissolved in 20 ml of anhydrous ethanol whereupon 114 mg (2,9 millimoles) of sodium borohydride are added under cooling with icecold water in portions. The addition having been completed the reaction mixture is stirred at room temperature overnight and thereafter the excess of sodium borohydride is decomposed with acetone. The ethanol solution is evaporated, the residue is dissolved in 15 ml of chloroform, washed with 5 ml of water and 5 ml of a saturated sodium chloride solution, dried over sodium sulfate and evaporated. The crude residue is purified by chromatography on silica and elution with a 1:1 mixture of n-hexane and ethyl acetate. Thus 198 mg of the desired compound are obtained in the form of a thick oil, yield 83%.
$R_f$ = 0.4 (1 : 1 mixture of n-hexane and ethylacetate).
$IR_{(film)}$: 3400, 1600, 1530, 1500, 1215, 1040, 820 $cm^{-1}$.

Example 10

(±)-1α-[Nitro]-2β-[1'-(fluoro)-phen-4'-yl]-4β-[methanesulfonyloxy]-cyclohexane

50 mg (0.209 millimoles) of (±)-1α-[nitro]-2β-[1'-(fluoro)-phen-4'-yl]-cyclohexane-4β-ol prepared according to Example 9 are dissolved in 10 ml of dichloromethane. To the solution 60 mg (0.521 millimoles) of methanesulfonyl chloride and 1.4 mole of pyridine are added, the reaction mixture is stirred at room temperature overnight and evaporated. The residue is dissolved in 30 ml of dichloromethane, washed with 10 ml of water and 10 ml of a saturated sodium chloride solution, dried over sodium sulfate and evaporated. The crude product is purified by column chromatography and elution with a 1:1 mixture of n-hexane and ethyl acetate. Thus 40 mg of the desired compound are obtained, yield 60%.
$R_f$ = 0.7 (1 : 1 mixture of n-hexane and ethyl acetate).
Mp.: 141-143 °C.
$IR_{(KBr)}$: 1600, 1550, 1500, 1170, 940, 820 $cm^{-1}$.

Example 11

(±)-1α-[Amino]-2β-[1'-(fluoro)-phen-4'-yl]-4β-[methanesulfonyloxy]-cyclohexane

0.268 g (0.844 millimoles of (±)-1α-[nitro]-2β-[1'-(fluoro)-phen-4'-yl]-4β-[methanesulfonyloxy]-cyclohexane prepared according to Example 10 are dissolved in 20 ml of ethanol. To the solution 2.23 g (9.88 millimoles) of stannous(II)-chloride dihydrate are added, the reaction mixture is heated under boiling on a bath (temperature 120 °C) for 24 hours, whereupon the ethanol is distilled off. The residue is taken up in a small amount of water and the pH is adjusted to 9-10 by adding a saturated sodium carbonate solution. The precipitated product is filtered, thoroughly washed with 200 ml of dichloromethane and the aqueous phase

is extracted three times with 100 ml of dichloromethane each. The combined dichloromethane layers are washed with 50 ml of brine, dried over sodium sulfate and evaporated. The crude residue is purified by column chromatography and elution with a 1:1 mixture of benzene and methanol. Thus 180 mg of the desired compound are obtained in the form of a yellow oil, yield 74%.
$R_f$ = 0.3 (1:1 mixture of benzene and methanol)
$IR_{(film)}$: 3360, 1600, 1500, 1210, 1160, 920, 820 $cm^{-1}$.

Example 12

(±)-endo-2β-[1'-(Fluoro)-phen-4'-yl]-7α-azabicyclo[2.2.1]heptane

100 mg (0.348 millimoles) of (±)-1α-[amino]-2β-[1'-(fluoro)-phen-4'-yl]-4β-[methanesulfonyloxy]-cyclohexane prepared according to Example 11 are dissolved in 10 ml of anhydrous toluene. The solution is heated to boiling on a bath (temperature 120°C) overnight under stirring. The toluene is distilled off, the residue is taken up in 20 ml of dichlormethane and the pH is adjusted to 10 by adding a saturated sodium carbonate solution. The phases are separated, the aqueous layer is extracted with 20 ml of dichloromethane, the combined organic layers are extracted with 10 ml of water and 10 ml of a saturated sodium chloride solution, dried over sodium sulfate and evaporated. The residue is purified by column chromatography and elution with a 1:1 mixture of benzene and methanol. Thus 50 mg of the desired compound are obtained in the form of a yellow oil, yield 75%.
$R_f$ = 0.15 (1:1 mixture of benzene and methanol).
$IR_{(film)}$: 3400, 3240, 1600, 1520, 1240, 820 $cm^{-1}$.

Example 13

(±)-endo-7-[Acetyl]-2β-[1'-(fluoro)-phen-4'-yl]-7α-azabicyclo[2.2.1]heptane

22 mg (0.115 millimoles) of (±)-endo-2β-[1'-(fluoro)-phen-4'-yl]-7-azabicyclo[2.2.1]heptane prepared according to Example 12 are dissolved in 10 ml of dichloromethane, whereupon 0.1 ml of pyridine and 57 mg (0.575 millimoles) of acetic anhydride are added. The reaction mixture is stirred at room temperature for 6 hours and evaporated. The residue is taken up in 20 ml of dichloromethane, washed with 2 ml of water and 5 ml of brine and evaporated. The crude residue is purified by column chromatography and elution with a 1:2 mixture of n-hexane and ethyl acetate. Thus 20 mg of the desired compound are obtained in the form of a yellow oil,
yield 74%.
$R_f$ = 0.2 (n-hexane: ethyl acetate = 1:2).

Example 14

1-[1'-(Chloro)-phen-4'-yl]-3-[oxo]-6-[nitro]-hexa-1-ene

2.8 g (19.9 millimoles) of 4-(chloro)-benzaldehyde are dissolved in 100 ml of anhydrous dichloromethane, whereupon 10.0 g (25.8 millimoles) of [5-(nitro)-pentane-2-one]-triphenyl-phosphorane prepared according to the part of Example 1 regarding the preparation of its starting substances are added at a bath temperature of 70°C within 24 hours under stirring. The reaction having been completed the dichloromethane solution is washed with 50 ml of water and 50 ml of a saturated sodium chloride solution, dried over sodium sulfate and evaporated. The crude residue is purified by column chromatography and elution with a 3:1 mixture of n-hexane and ethyl acetate. Thus 4.0 g of the desired compound are obtained in the form of a thick oil, yield 80%. $R_f$ = 0.4 (3:2 mixture of n-hexane and ethyl acetate).
$IR_{(film)}$: 1680, 1605, 1555, 1490, 1090, 1040, 980, 805 $cm^{-1}$.

Example 15

(±)-1α-[Nitro]-2β-[1'-(chloro)-phen-4'-yl]-cyclohexane-4-one

2.5 g (10 millimoles) of 1-[1'-(chloro)-phen-4'-yl]-3-[oxo]-6-[nitro]-hexa-1-ene prepared according to Example 14 are dissolved in 80 ml of anhydrous tetrahydrofurane, whereupon 4.46 g (99 millimoles) of potassium fluoride precipitated on aluminum oxide containing 13 mmoles of potassium fluoride are added

and the reaction mixture is stirred at room temperature for 24 hours. The solid product is removed by filtration and the tetrahydrofurane is distilled off. The crude residue is purified by column chromatography and elution with a 3:1 mixture of n-hexane and ethyl acetate. Thus 1.7 g of the desired compound are obtained, yield 68%, mp.: 97-104 °C.
$R_f$ = 0.2 (1:3 mixture of ethyl acetate and n-hexane).
$IR_{(KBr)}$: 1700, 1600, 1530, 1480, 1230, 1000, 820 $cm^{-1}$.

Example 16

(±)-1α-[Nitro]-2β-[1'-(chloro)-phen-4'-yl]-cyclohexane-4β-ol

1.2 g (4.73 millimoles) of (±)-1α-[nitro]-2β-[1'-(chloro)-phen-4'-yl]-cyclohexane-4-one prepared according to Example 15 are dissolved in 50 ml of anhydrous ethanol and 560 mg (14.2 millimoles) of sodium borohydride are added under cooling with icecold water in small portions. The addition having been completed the reaction mixture is stirred at room temperature overnight, whereupon the excess of borohydride is decomposed by adding acetone. The ethanolic solution is evaporated, the residue dissolved in 50 ml of chloroform, the organic phase is washed with 15 ml of water and 15 ml of brine, dried over sodium sulfate and evaporated. The crude residue is purified by column chromatography and elution with a 1:1 mixture of n-hexane and ethyl acetate. Thus 626 mg of the desired compound are obtained, yield 52%, mp.: 116-119 °C.
$R_f$ = 0.4 (1:1 mixture of n-hexane and ethyl acetate).
$IR_{(KBr)}$: 3380, 1550, 1500, 1380, 1060, 830 $cm^{-1}$.

Example 17

(±)-1α-[Nitro]-2β-[1'-(chloro)-phen-4'-yl]-4β-[methanesulfonyloxy]-cyclohexane

0.616 g (2.4 millimoles) of (±)-1α-[nitro]-2β-[1'-(chloro)-phen-4'-yl]-cyclohexane-4β-ol prepared according to Example 16 are dissolved in 20 ml of dichloromethane. To the solution 688 mg (5.98 millimoles) of methanesulfonyl chloride and 16.2 ml of pyridine are added. The reaction mixture is stirred at room temperature overnight and evaporated. The residue is dissolved in 30 ml of dichloromethane, washed with 10 ml of water and 10 ml of a saturated sodium chloride solution, dried over sodium sulphate and evaporated. The crude product is purified by column chromatography and elution with a 1:1 mixture of n-hexane and ethyl acetate. Thus 466 mg of the desired compound are obtained, yield 58%, mp.: 121-122 °C.
$IR_{(KBr)}$: 1560, 1540, 1500, 1180, 950, 850 $cm^{-1}$.
$R_f$ = 0.6 (1:1 mixture of n-hexane and ethyl acetate).

Example 18

(±)-1α-[Amino]-2β-[1'-(chloro)-phen-4'-yl]-4β-[methanesulfonyloxy]-cyclohexane

0.3 g (0.898 millimoles) of (±)-1α-[nitro]-2β-[1'-(chloro)-phen-4'-yl]-4β-[methanesulfonyloxy]-cyclohexane prepared according to Example 17 are dissolved in 20 ml of ethanol, whereupon 2.23 g (9.88 millimoles) of stannous(II)-chloride dihydrate are added and the reaction mixture is heated to boiling at a bath temperature of 120 °C for 24 hours. The ethanol is distilled off, the residue is taken up in a small amount of water and the pH is adjusted to 9-10 by adding a saturated sodium carbonate solution. The precipitated product is filtered, thoroughly washed with 200 ml of dichloromethane, the aqueous layer is extracted three times with 100 ml of dichloromethane each, the combined organic layers are washed with 50 ml of water and 50 ml of a saturated sodium chloride solution, dried over sodium sulfate and evaporated. The crude residue is purified by column chromatography and elution with a 1:1 mixture of benzene and methanol. Thus 150 mg of the desired compound are obtained, yield 55%,
mp.: 151-156 °C.
$IR_{(KBr)}$: 3360, 1600, 1510, 1210, 1170, 920, 820 $cm^{-1}$.
$R_f$ = 0.3 (1:1 mixture of benzene and methanol).

Example 19

(±)-endo-2β-[1'-(Chloro)-phen-4'-yl]-7α-azabicyclo[2.2.1]heptane

100 mg (0.33 millimoles) of (±)-endo-1α-[amino]-2β-[1'-(chloro)-phen-4'-yl]-4β-[methanesulfonyloxy]-cyclohexaneprepared according to Example 18 are dissolved in 10 ml of anhydrous toluene and the solution is heated to boiling (bath temperature 120 ° C) overnight under stirring. The toluene is removed, the residue is taken up in 20 ml of dichloromethane and the pH is adjusted to 9-10 by adding a saturated sodium carbonate solution. The layers are separated, the aqueous phase is extracted with 20 ml of dichloromethane, the combined organic layers are washed with 10 ml of water and 10 ml of a saturated sodium chloride solution, dried over sodium sulfate and evaporated. The residue is purified by column chromatography and elution with a 1:1 mixture of benzene and methanol. Thus 40 mg of the desired compound are obtained in the form of a yellow oil, yield 59%.
$IR_{(film)}$: 3350, 3240, 1600, 1520, 1230, 820 $cm^{-1}$.
$R_f$ = 0.15 (1:1 mixture of benzene and methanol).

Example 20

(±)-endo-7-[Acetyl]-2β-[1'-(chloro)-phen-4'-yl]-7α-azabicyclo[2.2.1]heptane

20 mg (0.096 millimoles) of (±)-endo-2β-[1'-(chloro)-phen-4'-yl]-7α-azabicyclo[2.2.1]heptane prepared according to Example 19 are dissolved in 10 ml of dichloromethane, whereupon 0.1 ml of pyridine and 48 mg (0.48 millimoles) of acetic anhydride are added. The reaction mixture is stirred at room temperature for 6 hours and evaporated. The residue is taken up in 20 ml of dichloromethane, washed with 2 ml of water and 5 ml of a saturated sodium chloride solution and evaporated. The crude product is purified by column chromatography and elution with a 1:2 mixture of n-hexane and ethyl acetate. Thus 15 mg of the desired compound are obtained in the form of a yellow oil, yield 62%.
$R_f$ = 0.2 (hexane: ethylacetate = 1:2).

Example 21

1-[6'-(Methoxy)-pyrid-3'-yl]-3-[oxo]-6-[nitro]-hexa-1-ene

18.25 g (46.69 millimoles) of [5-(nitro)-pentane-2-one]-triphenyl-phosphorane prepared according to the part of Example 1 regarding the preparation of its starting substances are dissolved in 500 ml of anhydrous toluene, whereupon 5 g (36.5 millimoles) of 6-(methoxy)-pyrid-3-yl-carboxaldehyde are added and the reaction mixture is heated to boiling under argon for 20 hours under stirring. After cooling the reaction mixture is washed with 150 ml of water and 150 ml of a saturated sodium chloride solution, dried and evaporated. The residue is purified by column chromatography and elution with a 50:1 mixture of chloroform and methanol. Thus 7.4 g of the desired compound are obtained, yield 81.6%, mp.: 58-60 ° C.
$R_f$ = 0.7.
$IR_{(KBr)}$: 1670, 1650, 1590, 1540, 1430, 1280, 1110 $cm^{-1}$.

Example 22

(±)-1α-[Nitro]-2β-[6'-(methoxy)-pyrid-3'-yl]-cyclohexane-4-one

5.0 g (20 millimoles) of 1-[6'-(methoxy)-pyrid-3'-yl]-3-[oxo]-6-[nitro]-hexa-1-ene prepared according to Example 21 are dissolved in 200 ml of anhydrous tetrahydrofurane, whereupon 8.9 g (198.9 millimoles) of potassium fluoride precipitated on aluminum oxide containing 40 mmoles of potassium fluoride are added and the reaction mixture is stirred at room temperature overnight. The solid precipitate is filtered and washed with 500 ml of ethyl acetate. The combined filtrates are dried over magnesium sulfate and evaporated. The residue is purified by chromatography on a silica column and elution with a 3:1 mixture of chloroform and methanol. Thus 2.45 g of the desired compound are obtained, yield 49%.
$R_f$ = 0.7.
$IR_{(KBr)}$: 1710, 1600, 1560, 1540, 1490, 1290 $cm^{-1}$.

Example 23

(±)-1α-[Nitro]-2β-[6'-(methoxy)-pyrid-3'-yl]-cyclohexane-4β-ol

3.0 g (12 millimoles) of (±)-1α-[nitro]-2β-[6'-(methoxy)-pyrid-3'-yl]-cyclohexane-4-one prepared according to Example 22 are dissolved in 150 ml of anhydrous methanol and 1.4 g (37 millimoles) of sodium borohydride are added at 0 °C in small portions within about 3 hours. The excess of the reducing agent is decomposed by careful dropwise addition of acetone and the reaction mixture is evaporated in vacuo. The residue is dissolved in the mixture of 100 ml of water and 200 ml of dichloromethane, the layers are separated. The combined organic layers are washed twice with 100 ml of water each and 100 ml of a saturated sodium chloride solution, dried over magnesium sulfate and evaporated. The solid residue is purified by chromatography on a silica column and elution with a 2:1 mixture of benzene and acetone ($R_f$ = 0.60). Thus 2 g of the pure desired compound are obtained, yield 66%, mp.: 143-144 °C.
$IR_{(KBr)}$: 3410, 1600, 1560, 1540, 1480, 1400, 1280, 1060, 1020 $cm^{-1}$.

Example 24

(±)-1α-[Nitro]-2β-[6'-(methoxy)-pyrid-3'-yl]-4β-[methanesulfonyloxy]-cyclohexane

2.0 g (8 millimoles) of (±)-1α-[nitro]-2β-[6'-(methoxy)-pyrid-3'-yl]-cyclohexane-4β-ol prepared according to Example 23 are dissolved in a mixture of 28 ml of dichloromethane and 53 ml of anhydrous pyridine, whereupon 1.55 ml (19.6 millimoles) of methanesulfonyl chloride are added dropwise under cooling with icecold water and the reaction mixture is stirred at room temperature for an hour. The solvent is evaporated, the residue is dissolved in a mixture of 100 ml of chloroform and 50 ml of a 10% by weight sodium carbonate solution and the layers are separated. The aqueous phase is extracted three times with 50 ml of chloroform each. The combined organic phases are washed three times with 100 ml of water each and 150 ml of a saturated sodium chloride solution, dried and evaporated. The residue is purified by chromatography on a silica column and elution with a 20:1 mixture of chloroform and methanol, $R_f$ = 0.7. Thus 2 g of the desired pure compound are obtained, yield 76%,
mp.: 131-132 °C.
$IR_{(KBr)}$: 1600, 1560, 1540, 1525, 1480, 1340, 1280, 1170, 940 $cm^{-1}$.

Example 25

(±)-1α-[Amino]-2β-[6'-(methoxy)-pyrid-3'-yl]-4β-[methanesulfonyloxy]-cyclohexane

1.0 g (3.03 millimoles) of (±)-1α-[nitro]-2β-[6'-(methoxy)-pyrid-3'-yl]-4β-[methanesulfonyloxy]-cyclohexane prepared according to Example 24 are dissolved in a mixture of 100 ml of anhydrous methanol and 1.33 ml of a 5 M hydrochloric dioxane solution (containing 6.6 millimoles of hydrogen chloride). The solution is hydrogenated in the presence of 1.0 g of a 10% by weight palladium/charcoal catalyst. The calculated amount of hydrogen (218 ml) having been taken up (48 hours) the catalyst is filtered, washed, the combined filtrates are evaporated. The residue is dissolved in a mixture of 20 ml of dichloromethane and 20 ml of a 5% by weight sodium carbonate solution, the mixture is thoroughly shaken and the two phases are separated. The aqueous layer is extracted three times with 20 ml of dichloromethane each, the combined organic layers are washed with 50 ml of a saturated sodium chloride solution, dried and evaporated. The residue is purified by chromatography on a silica column and elution with a 4:1 mixture of chloroform and methanol, $R_f$ = 0.3. Thus 0.7 g of the desired compound are obtained, yield 90%, mp.: 103-104 °C.
$IR_{(KBr)}$: 3380, 1600, 1560, 1480, 1330, 1160, 930 $cm^{-1}$.

Example 26

(±)-endo-2β-[6'-(Methoxy)-pyrid-3'-yl]-7α-azabicyclo[2.2.1]heptane

2.8 g (8.3 millimoles) of (±)-1α-[amino]-2β-[6'-(methoxy)-pyrid-3'-yl]-4β-[methanesulfonyloxy]-cyclohexane prepared according to Example 25 are dissolved in 350 ml of anhydrous toluene. The reaction mixture is heated to boiling under argon overnight. After cooling 250 ml of a 5% by weight sodium hydroxide solution are added, the mixture is thoroughly shaken and the layers are separated. The aqueous phase is extracted three times with 100 ml of dichloromethane each. The combined organic phases are washed

twice with 100 ml of water each and 100 ml of a saturated sodium chloride solution, dried over magnesium sulfate and evaporated. Thus 1.5 g of the desired compound are obtained in the form of a faint yellow oil, yield 78.7%.
$IR_{(film)}$: 3400, 3240, 1600, 1560, 1480, 1290, 1030 $cm^{-1}$.

Example 27

(±)-endo-7-[Acetyl]-2β-[6'-(methoxy)-pyrid-3'-yl]-7α-azabicyclo[2.2.1]heptane

1.4 g (6.8 millimoles) of (±)-endo-2β-[6'-(methoxy)-pyrid-3'-yl]-7α-azabicyclo[2.2.1]heptane prepared according to Example 26 are dissolved in 15 ml of anhydrous pyridine under cooling with icecold water, whereupon 3.3 ml (34.8 millimoles) of acetic anhydride are added. The reaction mixture is stirred at room temperature for 2 hours and then evaporated to dryness. The residue is admixed with ice, whereupon 50 ml of a 5% by weight sodium carbonate solution and 50 ml of dichloromethane are added. The mixture is thoroughly shaken, the layers are separated and the aqueous layer is extracted three times with 50 ml of dichloromethane each. The combined organic layers are washed twice with 50 ml of water each and 50 ml of a saturated sodium chloride solution, dried over magnesium sulfate and evaporated. The residue is purified by chromatography on a silica column and elution with a 1:1 mixture of chloroform and acetone. Thus 1.6 g of the desired compound are obtained in the form of a faint yellow oil, yield 94%.
$IR_{(film)}$: 1700, 1640, 1480, 1280, 1020 $cm^{-1}$.

Example 28

1-[Pyrid-3'-yl]-3-[oxo]-6-[nitro]-hexa-1-ene

To a solution of 14.2 g (0.036 mole) of [5-(nitro)-pentane-2-one]-triphenyl-phosphorane prepared according to the part of Example 1 regarding the preparation of its starting substances in 400 ml of anhydrous dichloromethane a solution of 3.0 g (0.028 mole) of pyrid-3-yl-carboxaldehyde in 100 ml of anhydrous dichloromethane is added. The reaction mixture is heated to boiling under argon for 16 hours and then cooled, whereupon the dichloromethane solution is washed three times with 150 ml of water each and 150 ml of a saturated sodium chloride solution, dried over calcium chloride and evaporated. The solid residue is purified by chromatography on a silica column and elution with a 3:1 mixture of chloroform and acetone. Thus 5.0 g of the desired pure compound are obtained, yield 81%, mp.: 61-63 °C.
$R_f$ = 0.5.
$IR_{(KBr)}$: 1700, 1680, 1620, 1550 $cm^{-1}$.

Example 29

(±)-1α[Nitro]-2β-[pyrid-3'-yl]-cyclohexane-4-one

5.0 g (0.0227 mole) of 1-[pyrid-3'-yl]-3-[oxo]-6-[nitro]-hexa-1-ene prepared according to Example 28 are dissolved in 230 ml of anhydrous tetrahydrofurane, whereupon 10.2 g (0.228 mole) of potassium fluoride precipitated on aluminum oxide containing 0.03 mole of potassium fluoride are added and the reaction mixture is heated to boiling at room temperature overnight. The solid product is filtered and washed with 500 ml of ethyl acetate. The combined filtrates are dried over calcium chloride and evaporated. The residue is purified by chromatography on a silica column and elution with a 3:1 mixture of chloroform and acetone. Thus 2.7 g of the pure desired compound are obtained, yield 54%,
mp.: 108-110 °C, $R_f$ = 0.25.

$IR_{(CHCl_3)}$:

1720, 1580, 1550 $cm^{-1}$.

Example 30

(±)-1α-[Nitro]-2β-[pyrid-3'-yl]-cyclohexane-4β-ol

2.3 g (0.0104 mole) of (±)-1α-[nitro]-2β-[pyrid-3'-yl]-cyclohexane-4-one prepared according to Example 29 are dissolved in 200 ml of anhydrous ethanol, whereupon at 0 °C in small portions 1.2 g (0.0317 mole) of sodium borohydride are added within about three hours. The excess of the reducing agent is decomposed by careful addition of acetone, whereupon the reaction mixture is evaporated in vacuo. The dry residue is dissolved in a mixture of 100 ml of water and 200 ml of dichloromethane. The mixture is thoroughly shaken and the layers are separated. The combined organic layers are washed twice with 200 ml of water each and 200 ml of a saturated sodium chloride solution, dried over calcium chloride and evaporated. The residue is purified by chromatography on a silica column and eluted with a 10:1 mixture of chloroform and methanol. Thus 1.8 g of the pure product are obtained, yield 79%, mp.: 146-150 °C.
$R_f$ = 0.29.
$IR_{(KBr)}$: 3300, 1550, 1070 $cm^{-1}$.

Example 31

(±)-1α-[Nitro]-2β-[pyrid-3'-yl]-4β-[methanesulfonyloxy]-cyclohexane

2.6 g (0.027 millimole) of (±)-1α-[nitro]-2β-[pyrid-3'-yl]-cyclohexane-4β-ol prepared according to Example 30 are dissolved in a mixture of 40 ml of dichloromethane and 30 ml of pyridine, whereupon 2.3 ml (0.0292 mole) of methanesulfonyl chloride are added under cooling with icecold water and the reaction mixture is stirred at room temperature overnight. Next morning the solvent is distilled off in vacuo, the dry residue is dissolved in a mixture of 100 ml of chloroform and 50 ml of a 10% by weight sodium carbonate solution. The mixture is thoroughly shaken and the layers are separated. The aqueous phase is extracted three times with 50 ml of chloroform each, the combined organic extracts are washed three times with 100 ml of water each and 100 ml of a saturated sodium chloride solution, dried over calcium chloride and evaporated. The solid residue is purified by chromatography on a silica column and elution with a 1:1 mixture of acetone and chloroform. Thus 3.1 g of the desired pure product are obtained, yield 87%, mp.: 170-172 °C, $R_f$ = 0.45.
$IR_{(KBr)}$: 1620, 1550, 1340, 1180, 940 $cm^{-1}$.

Example 32

(±)-1α-[Amino]-2β-[pyrid-3'-yl]-4β-[methanesulfonyloxy]-cyclohexane

1.0 g (0.00333 mole) of (±)-1α-[nitro]-2β-[pyrid-3'-yl]-4β-[methanesulfonyloxy]-cyclohexane prepared according to Example 31 are dissolved in a mixture of 100 ml of methanol and 1.33 ml of a 5 M hydrochloric dioxane solution (containing 0.00666 mole of hydrogen chloride) and the solution is hydrogenated in the presence of 1.0 g of 10% by weight palladium/charcoal catalyst. The calculated amount of hydrogen having been taken up (8-10 hours) the catalyst is filtered off and washed. The combined filtrates are evaporated. The dry residue (colourless foam) is dissolved in a mixture of 20 ml of dichloromethane and 20 ml of 5% by weight sodium carbonate solution. The mixture is thoroughly shaken and the two layers are separated. The aqueous phase is extracted five times with 20 ml of dichloromethane each. The combined organic phases are washed with 50 ml of a saturated sodium chloride solution, dried over calcium chloride and evaporated. Thus 750 mg of the desired compound are obtained in the form of a faint yellow oil, yield 83%, $R_f$ = 0.31.

$$IR_{(CHCl_3)}:$$

3400, 1190, 1050 $cm^{-1}$.

Example 33

(±)-endo-2β-[Pyrid-3'-yl]-7α-azabicyclo[2.2.1]heptane (±)-Dechloro-epi-epibatidine

300 mg (1.11 mole) of (±)-1α-[amino]-2β-[pyrid-3'-yl]-4β-[methanesulfonyloxy]-cyclohexane prepared according to Example 32 are dissolved in 40 ml of anhydrous toluene and the reaction mixture is heated to boiling under argon overnight. The reaction mixture is cooled, 25 ml of a 5% by weight sodium hydroxide solution are added, the layers are thoroughly shaken and then separated. The aqueous phase is extracted 10 times with 20 ml of dichloromethane each. The combined organic layers are washed twice with 50 ml of water each and 50 ml of a saturated sodium chloride solution, dried over magnesium sulfate and evaporated. Thus 93 mg of the desired compound are obtained in the form of a faint yellow oil, yield 48%, $R_f$ = 0.1.
(Methanol : benzene = 1:1).

Example 34

(±)-endo-7-[Acetyl]-2β-[pyrid-3'-yl]-7α-azabicyclo[2.2.1]heptane

384 mg (2.20 millimoles) of (±)-endo-2β-[pyrid-3'-yl]-7α-azabicyclo[2.2.1]heptane prepared according to Example 33 are dissolved in 5 ml of anhydrous pyridine, whereupon 1.1 ml (11.6 millimoles) of acetic anhydride are added under cooling with icecold water. The reaction mixture is stirred at room temperature overnight, whereupon the solution is evaporated to dryness. The residue is admixed with ice, 10 ml of a 5% by weight sodium carbonate solution and 10 ml of dichloromethane are added. The mixture is thoroughly shaken, the layers are separated, the aqueous phase is extracted three times with 10 ml of dichloromethane each. The combined organic extracts are washed twice with 10 ml of water each and 10 ml of a saturated sodium chloride solution, washed over magnesium sulfate and evaporated. Thus 223 mg of the desired compound are obtained in the form of a faint yellow oil, yield 47%, $R_f$ = 0.23
(chloroform : acetone = 1 : 1).
$IR_{(film)}$: 1700, 1640, 1440 $cm^{-1}$.

Example 35

(±)-1α-[Nitro]-2β-[6'-(chloro)-pyrid-3'-yl]-cyclohexane-4β-ol

A) (-)-1α-[Nitro]-2β-[6'-(chloro)-pyrid-3'-yl]-cyclohexane-4β-yl-carbonic acid menthyl ester

1.536 g (6 millimoles) of racemic (±)-1α-[nitro]-2β-[6'-(chloro)-pyrid-3'-yl]-cyclohexane-4β-ol prepared according to Example 3 are dissolved in a mixture of 30 ml of anhydrous dichloromethane and 1.4 ml of pyridine at room temperature. To the solution 3.0 ml (14 millimoles) of (-)-menthyl-chloro-formiate (Aldrich 24,530-5) are added dropwise. The reaction mixture is stirred at room temperature for 6 hours, whereupon further 0.2 ml of the reactant is added. The reaction mixture is allowed to stand overnight and then evaporated to dryness in vacuo. The residue is dissolved in a mixture of 60 ml of chloroform and 5 ml of water. The pH of the aqueous layer is adjusted to 9 with a 5% by weight sodium hydrogen carbonate solution. The layers are separated, the chloroform solution is washed three times with 20 ml of water each, dried over sodium sulfate, filtered and evaporated in vacuo. The residue is crystallized from 100 ml of methanol under clarification with charcoal. Thus 1.2 g of the desired compound are obtained in the form of white crystals,
yield 45.8%, mp.: 98-100 °C, $[\alpha]_D^{25}$ = -56.0 ° (c = 0.5, chloroform).

The product thus obtained is further purified by column chromatography (Merck 9385 silicagel, water pump vacuo; eluent = benzol : ethyl acetate = 19:1). The fractions containing the desired compound are evaporated. Thus 630 mg of an oil are obtained which is crystallized from methanol. Thus 280 mg of the desired compound are obtained, mp.: 183-184 °C, $[\alpha]_D^{25}$ = -36.7 ° (c = 0.5 chloroform).

B) (+)-1α-[Nitro]-2β-[6'-(chloro)-pyrid-3'-yl]-cyclohexane-4β-ol

100 mg of the above crystalline substance (-)-1α-[nitro]-2β-[6'-(chloro)-pyrid-3'-yl]-cyclohexane-4β-yl-carbonic acid menthyl ester {$[\alpha]_D^{25}$ = -36.7 (c = 0.5 chloroform)} are dissolved in a mixture of 20 ml of 10% by weight sulfuric acid and 20 ml of ethanol and the solution is heated to boiling for 24 hours,

whereupon the ethanol is removed in vacuo. To the aqueous residue about 30 ml of benzene are added and the mixture is evaporated again to dryness in vacuo. This operation is carried out five or six times. The residue is suspended in 30 ml of chloroform and the pH is adjusted to a value of about 10 by adding a concentrated ammonium hydroxide solution. The layers are separated, the chloroform solution is washed with water, dried over sodium sulfate, filtered and evaporated in vacuo. The residue is purified by column chromatography (Merck 9385 silicagel, water pump vacuo; eluent : chloroform :
: methanol = 20:1). The fractions containing the desired compound are combined and evaporated. The residue is crystallized from ether. Thus 32 mg of the desired compound are obtained, mp.: 190-194 °C, $[\alpha]_D^{25}$ = +63.9° (c = 0.5, chloroform).

Example 36

(+)-epi-epibatidine and (-)-epi-epibatidine

1.00 g (5 millimoles) of racemic epi-epibatidine prepared according to Example 6 are dissolved in 20 ml of hot acetone, whereupon a solution of 0.96 g (2.5 millimoles) of (-)-di-0,0'-p-toluoyl-L-tartaric acid in a mixture of 20 ml of acetone and 10 ml of water is added. The solution remains clear for some minutes but later the precipitation of crystals starts from the hot solution. The mixture is allowed to cool to room temperature and allowed to stand overnight in a refrigerator. Next morning the precipitated crystals are filtered, washed with a mixture of 5 ml of acetone and 1 ml of water and dried. Thus 1.053 g of the salt are obtained. Mp.: 188-190 °C, $[\alpha]_D^{25}$ = -56.9° (c = 0.5, methanol).

1.00 g of the above salt is dissolved in a hot mixture of 100 ml of ethanol and 5 ml of water and the solution is allowed to stand at room temperature for a week-end. The precipitated crystals are filtered, washed with a mixture of 5 ml of ethanol and 0.5 ml of water and dried. Thus 341 mg of the salt are obtained, mp.: 200-201 °C, $[\alpha]_D^{25}$ = -53.4° (c = 0.5, methanol).

300 mg of the above salt are suspended in a mixture of 120 ml of chloroform and 8 ml of water at room temperature and sufficient amount of a 1 molar sodium hydroxide solution (about 0.2-0.3 ml) is added to adjust the pH of the aqueous phase to 9-10. The layers are separated, the chloroform solution is washed twice with 8 ml of water each, dried over sodium sulfate, filtered and evaporated in vacuo. Thus 155 mg of an oily product are obtained, $[\alpha]_D^{25}$ = +36.1° (c = 0.5, methanol).

(-)-Epi-epibatidine

The first crystallization mother-lye obtained by the preparation of (+)-epi-epibatidine is evaporated to dryness in vacuo and the crystalline residue is made water-free by evaporation on a rotating evaporator several times. Thus 649 mg of the product are obtained,
mp.: 164-176 °C, $[\alpha]_D^{25}$ = -64.8° (c = 0.5, methanol).

200 mg of the above salt are suspended in a mixture of 80 ml of chloroform and 6 ml of water. The pH is adjusted to 9-10 by adding a 1 molar sodium hydroxide solution. The layers are separated, the chloroform solution is washed with water (about 10 ml), dried over sodium sulfate, filtered and evaporated to dryness. Thus 96 mg of an oily product are obtained, $[\alpha]_D^{25}$ = -17.2° (c = 0.5, methanol).

400 mg of the above salt {$[\alpha]_D^{25}$ = -64.8° (c = 0.5, methanol)} are treated with 2 ml of a 1 M sodium hydroxide solution in a mixture of 150 ml of chloroform and 10 ml of water. The reaction mixture is worked up. 160 mg of an oily product are obtained.
$[\alpha]_D^{25}$ = -17.2° (c = 0.5, methanol).

The oily crude product thus obtained (160 mg, 0.76 millimole) is dissolved in 3.2 ml of hot acetone, whereupon a solution of 153 mg (0.396 millimole) of (+)-di-0,0'-p-toluoyl-D-tartaric acid, 3.2 ml of acetone and 0.6 ml of water is added. The reaction mixture is allowed to cool to room temperature, allowed to stand for a few hours, the precipitated crystals are filtered, washed with some drops of aqueous acetone and dried. Thus 269 mg of the salt are obtained, mp.: 196-198 °C,
$[\alpha]_D^{25}$ = +63.4° (c = 0.5, methanol).

200 mg of the above salt are recrystallized from a mixture of 4.5 ml of ethanol and 0.5 ml of water. Thus 124 mg of the pure salt are obtained, mp.: 204-205 °C,
$[\alpha]_D^{25}$ = +60.3°, (c = 0.5 methanol).

100 mg of the salt thus obtained are treated with 2 ml of a 1 M sodium hydroxide solution in a mixture of 40 ml of chloroform and 4 ml of water. Thus 60 mg of an oily product are obtained, $[\alpha]_D^{25}$ = -40.3° (c = 0.5, methanol).

Example 37

1-[1',3'-Di-(chloro)-phen-4'-yl]-3-[oxo]-6-[nitro]-hexa-1-ene

17.5 g (100 millimoles) of 2,4-di-(chloro)-benzaldehyde are dissolved in 200 ml of anhydrous dichloromethane whereupon 50.8 g (130 millimoles) of [5-(nitro)-pentane-2-one]-triphenyl-phosphorane prepared according to the part of Example 1 regarding the preparation of its starting substances are added and the reaction mixture is stirred at a bath-temperature of 70-80 °C for 24 hours. The reaction having been completed the dichloromethane solution is washed with 50 ml of water and twice with 50 ml of a saturated sodium chloride solution each, dried over sodium sulfate, and evaporated. The crude product is purified by column chromatography and elution with a 3:1 mixture of n-hexane and ethyl acetate. Thus 17.9 g of the desired product are obtained in the form of a light yellow thick oil, yield 62%, $R_f$ = 0.4 (n-hexane : ethyl acetate = = 3 : 1).
$IR_{(film)}$: 1700, 1680, 1620, 1550, 1480, 1200, 1160 $cm^{-1}$.

Example 38

(±)-1α-[Nitro]-2β-[1',3'-di-(chloro)-phen-4'-yl]-cyclohexane-4-one

17.9 g (62.1 millimoles) of 1-[1',3'-di-(chloro)-phen-4'-yl]-3-[oxo]-6-[nitro]-hexa-1-ene prepared according to Example 37 are dissolved in 150 ml of anhydrous tetrahydrofurane, whereupon 28 g (622 millimoles) of potassium fluoride precipitated on aluminum oxide containing 82 mmoles of potassium fluoride are added and the reaction mixture is stirred at room temperature for 24 hours. The solid phase is removed by filtration, the tetrahydrofurane is distilled off, the residue is subjected to column chromatography and eluted with a 3:1 mixture of n-hexane and ethyl acetate. Thus 11.4 g of the crystalline desired compound are obtained, yield 63%, mp.: 127-129 °C,
$R_f$ = 0.25 (n-hexane : ethyl acetate = 3:1).
$IR_{(KBr)}$: 1720, 1600, 1550, 1380, 1120, 880 $cm^{-1}$.

Example 39

(±)-1α-[Nitro]-2β-[1',3'-di-(chloro)-phen-4'-yl]-cyclohexane-4β-ol

8.9 g (30.8 millimoles) of (±)-1α-[nitro]-2β-[1',3'-di-(chloro)-phen-4'-yl]-cyclohexane-4-one prepared according to Example 38 are dissolved in 300 ml of anhydrous ethanol, whereupon 3.7 g (97 millimoles) of sodium borohydride are added under cooling with icecold water and stirring in portions. The addition having been completed the reaction mixture is stirred for further 2 hours whereupon the excess of sodium borohydride is decomposed by adding acetone under cooling. The reaction mixture is evaporated, the residue is taken up in 200 ml of chloroform, the organic layer is washed twice with 50 ml of water each and 50 ml of a saturated sodium chloride solution, dried over sodium sulfate and evaporated. The crude product is purified by column chromatography and elution with a 1:1 mixture of n-hexane and ethyl acetate. Thus 5.8 g of the desired compound are obtained in the form of a yellow thick oil, yield 65%, $R_f$ = 0.3 (n-hexane : ethyl acetate = 1:1).
$IR_{(film)}$: 3400, 1600, 1550 $cm^{-1}$.

Example 40

(±)-1α-[Nitro]-2β-[1',3'-di-(chloro)-phen-4'-yl]-4β-[methanesulfonyloxy]-cyclohexane

5.8 g (20 millimoles) of (±)-1α-[nitro]-2β-[1',3'-di-(chloro)-phenyl]-cyclohexane-4β-ol prepared according to Example 39 are dissolved in 200 ml of anhydrous dichloromethane, whereupon 5.7 g (49 millimoles) of methanesulfonyl chloride and 50 ml of pyridine are added. The reaction mixture is stirred at room temperature for 3 hours and evaporated. To the residue 200 ml of dichloromethane are added, whereupon the pH is adjusted to 9-10 with a saturated sodium carbonate solution. The organic phase is separated, washed with 50 ml of water and 50 ml of a saturated sodium chloride solution, dried over sodium sulfate and evaporated. The crude product is subjected to column chromatography and elution with a 1:1 mixture of n-hexane and ethyl acetate. Thus 6.0 g of the desired compound are obtained in the form of a dark yellow thick oil, yield 81%. $R_f$ = 0.60 (n-hexane) : ethyl acetate = 1:1).

$IR_{(film)}$: 1600, 1550, 1500, 1160, 930, 820 $cm^{-1}$.

Example 41

(±)-1α-[Amino]-2β-[1',3'-di-(chloro)-phen-4'-yl]-4β-[methanesulfonyloxy]-cyclohexane

6.75 g (18.32 millimoles) of (±)-1α-[nitro]-2β-[1',3'-di-(chloro)-phen-4'-yl]-4β-[methanesulfonyloxy]-cyclohexane prepared according to Example 40 are dissolved in 300 ml of ethanol, whereupon 50 g (221 millimoles) of stannous(II)-chloride dihydrate are added and the reaction mixture is stirred at 110 °C for 10 hours. The ethanol is removed, the residue is dissolved in a small amount of water and the pH is adjusted to 9-10 with a saturated sodium carbonate solution. The precipitated product is filtered, washed thoroughly with 500 ml of dichloromethane, the aqueous layer is extracted three times with 50 ml of dichloromethane each. The combined organic layers are washed with 100 ml of water and 100 ml of a saturated sodium chloride solution, dried over sodium sulfate and evaporated. The crude product is used in the next step without purification, $R_f$ = 0.40
(chloroform : methanol = 1:1).
Yield 4.5 g (72%).

Example 42

(±)-endo-2β-[1',3'-Di-(chloro)-phen-4'-yl]-7α-azabicyclo[2.2.1]heptane

4.5 g (2.9 millimoles) of (±)-1α-[amino]-2β-[1',3'-di-(chloro)-phen-4'-yl]-4β-[methanesulfonyloxy]-cyclohexane prepared according to Example 41 are dissolved in 300 ml of anhydrous toluene and the reaction mixture is heated to boiling at 100 °C under stirring for 5 hours. The toluene is distilled off, the residue is dissolved in 200 ml of chloroform and the pH is adjusted to 9-10 with a saturated sodium carbonate solution. The layers are separated, the aqueous phase is extracted with 200 ml of chloroform, the combined organic solutions are washed with 50 ml of water and 50 ml of a saturated sodium chloride solution, dried over sodium sulfate and evaporated. The crude product is purified by column chromatography and eluted with a 1:1 mixture of chloroform and methanol. Thus 1.93 g of the desired compound are obtained in the form of a viscous yellow oil, yield 60%, $R_f$ = 0.15
(chloroform : methanol = 1:1).
$IR_{(film)}$: 3340, 3240, 1600, 1520, 1220, 830 $cm^{-1}$.

Example 43

1-[1',3'-Di-(methoxy)-phen-4'-yl]-3-[oxo]-6-[nitro]-hexa-1-ene

To a solution of 30.25 g (77.3 millimoles) of [5-(nitro)-pentane-2-one]-triphenyl-phosphorane prepared according to the part of Example 1 regarding the preparation of its starting substances in 700 ml of anhydrous toluene 10 g (60.17 millimoles) of 2,4-di-(methoxy)-benzaldehyde are added. The reaction mixture is refluxed under argon for 70 hours. After cooling the toluene solution is washed three times with 200 ml of water each and 150 ml of a saturated sodium chloride solution, dried over magnesium sulfate and evaporated. The residue is purified by column chromatography and eluted with a 5:2 mixture of n-hexane and ethyl acetate. Thus 11.2 g of the desired compound are obtained, yield 66.7%, $R_f$ = 0.5, mp.: 54-55 °C.

Example 44

(±)-1α-[Nitro]-2β-[1',3'-di-(methoxy)-phen-4'-yl]-cyclohexane-4-one

11.0 g (39.4 millimoles) of 1-[1',3'-di-(methoxy)-phen-4'-yl]-3-[oxo]-6-[nitro]-hexa-1-ene prepared according to Example 43 are dissolved in 330 ml of anhydrous tetrahydrofurane, whereupon 17.6 g (393.4 millimoles) of potassium fluoride containing 52 mmoles of potassium fluoride precipitated on aluminum oxide are added and the reaction mixture is stirred at room temperature for 48 hours. The solid product is removed by filtration and washed with 600 ml of ethyl acetate. The combined filtrates are evaporated. The residue is subjected to chromatography on a silica gel column and eluted with a 2:1 mixture of benzene and acetone. Thus 9.0 g of the pure desired compound are obtained as an oil, yield 81.8%, $R_f$ = 0.85.

Example 45

(±)-1α-[Nitro]-2β-[1',3'-di-(methoxy)-phen-4'-yl]-cyclohexane-4β-ol

1.0 g (3.58 millimoles) of (±)-1α-[nitro]-2β-[1',3'-di-(methoxy)-phen-4'-yl]-cyclohexane-4-one prepared according to Example 44 are dissolved in 50 ml of anhydrous methanol. The solution is cooled to 0-5 °C, 0.4 g (10.7 millimoles) of sodium borohydride are added in portions (about 1 hour) and the reaction mixture is stirred at 0-5 °C for an hour. The excess of sodium borohydride is decomposed with acetone and the mixture evaporated. The residue is dissolved in a mixture of 50 ml of water and 100 ml of dichloromethane, the mixture is thoroughly shaken and the layers are separated. The aqueous phase is extracted three times with 50 ml of dichloromethane each. The combined organic solutions are dried and evaporated. The residue is purified by column chromatography and elution with a 2:1 mixture of benzene and acetone. Thus 0.7 g of the desired pure product are obtained, yield 70%, $R_f$ = 0.6 (oil).

Example 46

(±)-1α-[Nitro]-2β-[1',3'-di-(methoxy)-phen-4'-yl]-4β-[methanesulfonyloxy]-cyclohexane

6.3 g (22.4 millimoles) of (±)-1α-[nitro]-2β-[1',3'-di-(methoxy)-phen-4'-yl]-cyclohexane-4β-ol prepared according to Example 45 are dissolved in a mixture of 90 ml of anhydrous dichloromethane and 180 ml of pyridine, whereupon 4.41 ml (55.9 millimoles) of methanesulfonyl chloride are added and the reaction mixture is stirred at room temperature overnight. The solvent is distilled off in vacuo, the residue is dissolved in a mixture of 200 ml of chloroform and 100 ml of a 10% by weight sodium carbonate solution. The mixture is thoroughly shaken and the layers are separated. The aqueous phase is extracted three times with 100 ml of chloroform each. The combined organic layers are washed with 3 x 100 ml of water and 100 ml of a saturated sodium chloride solution, dried and evaporated. The residue is purified by chromatography on a silica column and elution with a 20:1 mixture of chloroform and methanol. Thus 5.5 g of the desired compound are obtained, yield 69%,
$R_f$ = 0.8, mp.: 142-144 °C.

Example 47

(±)-1α-[Amino]-2β-[1',3'-di-(methoxy)-phen-4'-yl]-4β-[methanesulfonyloxy]-cyclohexane

1.1 g (3.06 millimoles) of (±)-1α-[nitro]-2β-[1',3'-di-(methoxy)-phen-4'-yl]-4β-[methanesulfonyloxy]-cyclohexaneprepared according to Example 46 are dissolved in a mixture of 150 ml of methanol and 4 ml of dioxane containing 20 mmoles of hydrochloric acid. The solution is hydrogenated in the presence of 1.1 g of a 10% by weight palladium charcoal catalyst. The calculated amount of hydrogen having been taken up (about 50 hours) the catalyst is filtered off and washed. The filtrate is evaporated. The residue is dissolved in a mixture of 20 ml of dichloromethane and 20 ml of a 5% by weight sodium carbonate solution, the mixture is thoroughly shaken and the layers are separated. The aqueous phase is extracted five times with 20 ml of dichloromethane each. The combined organic layers are dried and evaporated. The residue is purified by column chromatography and elution with a 3:1 mixture of chloroform and acetone. Thus 0.9 g of the desired compound are obtained in the form of an oil, yield 89%, $R_f$ = 0.16.

Example 48

(±)-endo-2β-[1',3'-Di-(methoxy)-phen-4'-yl]-7α-azabicyclo[2.2.1]heptane

2.1 g (6.38 millimoles) of (±)-1α-[amino]-2β-[1',3'-di-(methoxy)-phen-4'-yl]-4β-[methanesulfonyloxy]-cyclohexaneprepared according to Example 47 are dissolved in 260 ml of anhydrous toluene and the reaction mixture is refluxed under argon overnight. The mixture is cooled, 200 ml of a 5% by weight sodium hydroxide solution are added, the mixture is thoroughly shaken and the layers are separated. The aqueous phase is extracted five times with 100 ml of dichloromethane each. The combined organic solutions are dried over magnesium sulfate and evaporated. The residue is purified by chromatography on a silica column and elution with a 9:1 mixture of chloroform and methanol. Thus 950 mg of the desired compound are obtained in the form of a viscous oil, yield 63.8%, $R_f$ = 0.14.

## Claims

1. Epi-epibatidine derivatives of general formula

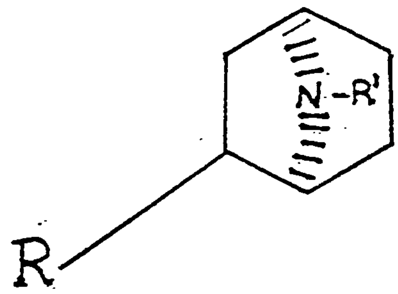

XVII,

wherein

R stands for a $C_{1-4}$-alkyl, $C_{2-4}$-alkenyl, $C_{2-4}$-alkinyl, $C_{3-7}$-cycloalkyl, aryl, heteroaryl, aryl-$C_{1-4}$-alkyl or heteroaryl-$C_{1-4}$-alkyl group, said groups optionally being substituted by 1 or more $C_{1-4}$-alkyl, $C_{2-4}$-alkenyl, $C_{2-4}$-alkinyl, $C_{3-7}$-cycloalkyl, aryl, heteroaryl, aryl-$C_{1-4}$-alkyl, heteroaryl-$C_{1-4}$-alkyl, hydroxy, $C_{1-4}$-alkoxy, phenoxy, halogen, halogeno-$C_{1-4}$-alkyl and/or amino, amido and/or sulfonamido substituent(s), optionally mono- or di-$C_{1-4}$-alkyl-, -$C_{2-4}$-alkenyl- and/or -$C_{2-4}$-alkinyl substituted, and

R' represents hydrogen or a $C_{1-4}$-alkyl, $C_{2-4}$-alkenyl, $C_{2-4}$-alkinyl, $C_{3-7}$-cycloalkyl, $C_{3-7}$-cycloalkenyl, $C_{3-7}$-cycloalkinyl, aryl -$C_{1-4}$-alkyl, aryl, heteroaryl, halogeno-$C_{1-4}$-alkyl, hydroxy-$C_{1-4}$-alkyl or, preferably $C_{1-4}$-aliphatic, aromatic or heterocyclic, acyl group with the proviso that,

if R' stands for hydrogen,

R is different from 6-(chloro)-pyrid-3-yl

as well as optically active forms and acid addition salts thereof.

2. Epi-epibatidine derivatives according to claim 1, characterized in that the $C_{1-4}$-alkyl group(s) which may be represented by R and/or R' and/or by which the group represented by R may be substituted is/are such having 1 or 2 carbon atom(s) and/or the $C_{2-4}$-alkenyl and/or $C_{2-4}$-alkinyl group(s) which may be represented by R and/or R' and/or by which the group represented by R may be substituted is/are such having 2 or 3 carbon atoms and/or the $C_{3-7}$-cycloalkyl group(s) which may be represented by R and/or R' and/or by which the group represented by R may be substituted is/are such having 5 to 7 carbon atoms and/or the $C_{3-7}$-cycloalkenyl or $C_{3-7}$-cycloalkinyl group which may be represented by R' is such having 5 to 7 carbon atoms and/or the alkyl part of the aryl-$C_{1-4}$-alkyl group(s) which may be represented by R and/or R' and/or by which the group represented by R may be substituted is/are such having 1 or 2 carbon atom(s) and/or the alkyl part of the heteroaryl-$C_{1-4}$-alkyl and/or halogeno-$C_{1-4}$-alkyl and/or mono- or di-$C_{1-4}$-alkyl substituted amino-, amido- and/or sulfonamido group(s) by which the group represented by R may be substituted is/are such having 1 or 2 carbon atom(s) and/or the $C_{2-4}$-alkenyl and/or $C_{2-4}$-alkinyl part of the mono- or di-$C_{2-4}$-alkenyl- and/or -$C_{2-4}$-alkinyl substituted amino-, amido- and/or sulfonamido group(s) by which the group represented by R may be substituted is/are such having 2 or 3 carbon atom(s) and/or the $C_{1-4}$-alkoxy group(s) by which the group represented by R may be substituted is/are such having 1 or 2 carbon atom(s) and/or the alkyl part of the halogeno-$C_{1-4}$-alkyl and/or hydroxy-$C_{1-4}$-alkyl group which may be represented by R' is such having 1 or 2 carbon atom(s) and/or the $C_{1-4}$-aliphatic acyl group which may be, represented by R' is such having 1 or 2 carbon atom(s) and/or the aryl group(s) which may be represented by R and/or R' and/or by which the group represented by R may be substituted is/are [a] phenyl and/or naphthyl group(s) and/or the aryl part of the aryl-$C_{1-4}$-alkyl group(s) which may be represented by R and/or R' and/or by which the group represented by R may be substituted is/are [a] phenyl and/or naphthyl group(s) and/or the heteroaryl group(s) which may be represented by R and/or R' and/or by which the group represented by R may be substituted is/are 5-to 7-membered in the heterocyclic ring (part), optionally with a benzene ring condensed to it/them, and/or such having 1 or more nitrogen, oxygen and/or sulfur atom(s) in the heterocylic ring (part) and/or the heteroaryl part of the heteroaryl-$C_{1-4}$-alkyl group(s) which may be represented by R and/or by which the group represented by R may be

substituted is/are 5- to 7-membered in the heterocyclic ring (part), optionally with a benzene ring condensed to it/them, and/or such having 1 or more nitrogen, oxygen and/or sulfur atom(s) in the hetero-cyclic ring (part) and/or the halogen atom(s) by which the group represented by R may be substituted is/are chlorine and/or fluorine and/or the halogen atom(s) of the halogeno-$C_{1-4}$-alkyl group-(s) which may be represented by R' and/or by which the group represented by R may be substituted is/are chlorine and/or fluorine and/or the aromatic ring of the aromatic acyl group(s), which may be represented by R' is a benzene or naphthalene ring and/or the heterocyclic ring of the heterocyclic acyl group(s) which may be represented by R' is 5- to 7-membered in the heterocyclic ring (part), optionally with an benzene ring condensed to it/them, and/or such having 1 or more nitrogen, oxygen and/or sulfur atom(s) in the heterocyclic ring (part) group.

**3.** Epi-epibatidine derivatives according to claim 1 or 2, characterized in that R stands for a phenyl or pyridyl group, optionally substituted by halogen or $C_{1-4}$-alkoxy.

**4.** Epi-epibatidine derivatives according to claims 1 to 3, characterized in that R stands for a 4-(fluoro)-phenyl, 4-(chloro)-phenyl, 6-(methoxy)-pyrid-3-yl, pyridyl, 1,3-di-(chloro)-phenyl, 1,3-di-(methoxy)-phenyl or 6-(ethoxy)-pyridyl group.

**5.** Epi-epibatidine derivatives according to claims 1 to 4, characterized in that R' stands for hydrogen or an acetyl group.

**6.** Process for preparing the epi-epibatidine derivatives according to claims 1 to 5 and epi-epibatidine of formula XVII in which R stands for a 6-(chloro)-pyrid-3-yl group and R' represents hydrogen, characterized by

a) cyclising a racemic or optically active substituted aminocyclohexane of general formula

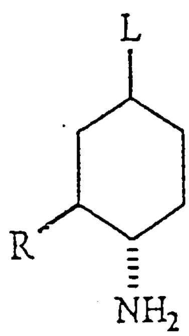

I,

wherein

L represents a leaving group and

R is as defined in claims 1 to 5 without exclusion of the 6-(chloro)-pyrid-3-yl group for the case of stopping after cyclisation to epi-epibatidine

or

b) reducing a racemic or optically active substituted nitrocyclohexane of general formula

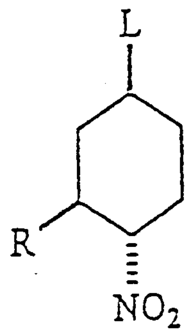

II,

wherein

L represents a leaving group and

R is as defined in claims 1 to 5 without exclusion of the 6-(chloro)-pyrid-3-yl group for the case of stopping after cyclisation to epi-epibatidine,

and cyclising the substituted nitrocyclohexane of general formula I thus obtained

and in a manner known per se, if desired, alkylating, alkenylating, alkinylating, cycloalkylating, cycloalkenylating, cycloalkinylating, arylating, heteroarylating or acylating the epi-epibatidine (derivative) of general formula XVII, wherein R' stands for hydrogen, thus obtained and/or, if desired, converting the epi-epibatidine (derivative) of general formula XVII, thus obtained into an acid addition salt thereof and/or, if desired, liberating from the acid addition salt of the epi-epibatidine (derivative) of general formula XVII thus obtained the free epi-epibatidine (derivative) of general formula XVII and/or converting the latter into another acid addition salt thereof and/or, if desired, resolving the racemic epi-epibatidine (derivative) of general formula XVII or an acid addition salt thereof thus obtained into the optically active isomers thereof and/or racemising the optically active isomers.

**7.** Process according to claim 6, characterized by carrying out the cyclisation under heating.

**8.** Process according to claim 6 or 7, characterized by carrying out the cyclisation in an aprotic solvent, particularly a halogenated hydrocarbon or an aromatic hydrocarbon, or a mixture thereof.

**9.** Process according to claim 6, 7 or 8, characterized by using benzene, toluene and/or xylene as aprotic solvent(s).

**10.** Process according to claims 6 to 9, characterized in using as starting material a substituted aminocyclohexane or nitrocyclohexane of general formula I or II, respectively, wherein L stands for a $C_{1-4}$-alkylsulfonyloxy or arylsulfonyloxy group, optionally substituted by [a] $C_{1-4}$-alkyl group(s) or halogen(s).

**11.** Process according to claims 6 to 10, characterized by using as starting material a substituted aminocyclohexane or nitrocyclohexane of general formula I or II, respectively, wherein L stands for a methanesulfonyloxy, p-toluenesulfonyloxy or p-bromo-phenylsulfonyloxy group.

**12.** Process according to claim 6 b), characterized by carrying out the reduction of the substituted nitrocyclohexane of general formula II by catalytic hydrogenation or chemical reduction.

**13.** Process according to claim 6 b) or 12, characterized by carrying out the catalytic hydrogenation in the presence of a palladium catalyst.

**14.** Process according to claim 6 b) or 12, characterized by carrying out the chemical reduction by Bechamps reduction or in glacial acetic acid with zinc or in hydrochloric acid with zinc, iron or tin or with stannous(II)-chloride.

**15.** Medicaments, characterized in that they contain 1 or more epi-epibatidine derivative(s) according to claims 1 to 5 and/or epi-epibatidine and/or acid addition salt(s) thereof as [an] active ingredient(s), advantageously in admixture with 1 or more inert solid and/or liquid pharmaceutical carrier(s) and/or auxiliary agent(s).

**16.** The use of the epi-epibatidine derivatives according to claims 1 to 5 and/or of epi-epibatidine and/or of acid addition salts thereof for the preparation of medicaments, particularly with analgesic activity.

**17.** Substituted aminocyclohexanes of general formula

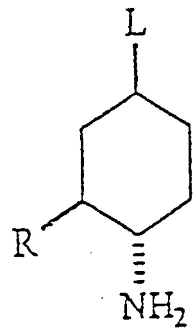

I,

wherein

L represents a leaving group and

R is as defined in claims 1 to 5 there being no exclusion of the 6-(chloro)-pyrid-3-yl group in view of epi-epibatidine.

**18.** Substituted nitrocyclohexanes of general formula

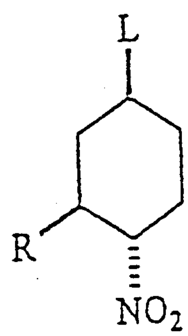

II,

wherein

L represents a leaving group and

R is as defined in claims 1 to 5 there being no exclusion of the 6-(chloro)-pyrid-3-yl group in view of epi-epibatidine.

**19.** Substituted 1-(nitro)-cyclohexane-4-ols of general formula

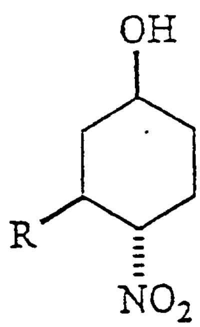

III,

wherein

R is as defined in claims 1 to 5 there being no exclusion of the 6-(chloro)-pyrid-3-yl group in view of epi-epibatidine.

**20.** Substituted 1-(nitro)-cyclohexane-4-ones of general formula

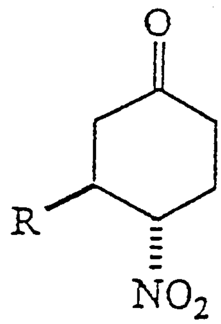

IV,

wherein

R is as defined in claims 1 to 5 there being no exclusion of the 6-(chloro)-pyrid-3-yl group in view of epi-epibatidine.

**21.** Substituted 3-(oxo)-6-(nitro)-hexa-1-enes of general formula

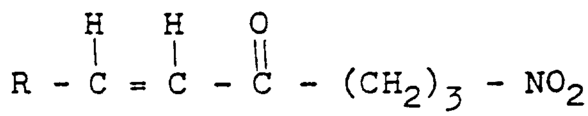

V,

wherein

R is as defined in claims 1 to 5 there being no exclusion of the 6-(chloro)-pyrid-3-yl group in view of epi-epibatidine.

European Patent Office

# EUROPEAN SEARCH REPORT

Application Number
EP 94 11 9481

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | CHEMICAL ABSTRACTS, vol. 117, no. 11, 14 September 1992, Columbus, Ohio, US; abstract no. 111485r, T. ASBEROM ET AL. 'Preparation of 4,5-cycloalkano-3-benzazepin-7-ols as dopaminergic D1 antagonists.' page 853 ; **CAS RN 136760-49-1* & WO-A-91 11437 (SCHERING CORPORATION) --- | 20 | C07D487/08 A61K31/44 |
| X | JOURNAL OF ORGANIC CHEMISTRY., vol.23, 1958, EASTON US pages 794 - 796 N.L. DRAKE ET AL. 'Polycyclic compounds containing nitrogen. II. hydroindoles.' *3-(2-ethoxyethyl)-4-nitrocyclohexanone: CAS RN 99189-98-7* --- | 20 | |
| X | JOURNAL OF ORGANIC CHEMISTRY., vol.39, no.10, 1974, EASTON US pages 1407 - 1410 M.E. LEWELLYN ET AL. 'Formation of a cyclohexane ring by condensation of a nitro ketone and an aldehyde.' *3-(2-furyl)-4-nitrocyclohexanone: CAS RN 51004-08-1* * page 1408, column 2 * --- | 20 | **TECHNICAL FIELDS SEARCHED (Int.Cl.6)** C07D A61K |
| X | INDIAN JOURNAL OF CHEMISTRY, vol.5, no.7, 1967 pages 344 - 346 J.S. BINDRA ET AL. '2-Substituted octahydrophenanthridines.' *Compounds II, VII: CAS RN 18123-22-3, 18123-28-9* * page 344, column 2 * --- | 19,20 | |
| | -/-- | | |

The present search report has been drawn up for all claims

| Place of search | Date of completion of the search | Examiner |
|---|---|---|
| THE HAGUE | 23 March 1995 | Bosma, P |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 11 9481

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | BIOORGANIC & MEDICINAL CHEMISTRY LETTERS, vol.3, no.12, 1993, OXFORD pages 2759 - 2764 T. LI ET AL. 'The analgesic effect of epibatidine and isomers.' --- | 1-3,5, 15,16 | |
| X,P | TETRAHEDRON LETTERS., vol.35, no.8, 21 February 1994, OXFORD GB pages 1299 - 1300 R.W. ABEN ET AL. 'Synthesis of endo-2-phenyl-7-azabicyclo(2.2.1)heptane via high pressure Diels-Alder reactions of pyrroles.' *CAS RN 155053-30-8* --- | 1-3,5 | |
| X,P | TETRAHEDRON LETTERS., vol.35, no.19, 9 May 1994, OXFORD GB pages 3171 - 3174 C. SZANTAY ET AL. 'A practical route to epibatidine' *CAS RN 152377-48-5, 156489-42-8, 156489-43-9, 156489-44-0* --- | 1-3,5, 15,16, 18,20,21 | TECHNICAL FIELDS SEARCHED (Int.Cl.6) |
| X,P | JOURNAL OF THE CHEMICAL SOCIETY, CHEMICAL COMMUNICATIONS, no.15, 17 August 1994, LETCHWORTH GB pages 1775 - 1776 S.Y. KO ET AL. 'The total synthesis of epibatidine' *compound 10* --- | 17 | |
| A,D | WO-A-93 18037 (THE UNITED STATES OF AMERICA, DEPARTMENT OF HEALTH AND HUMAN SERVICES) *scheme 1* --- -/-- | 1-16 | |

The present search report has been drawn up for all claims

| Place of search | Date of completion of the search | Examiner |
|---|---|---|
| THE HAGUE | 23 March 1995 | Bosma, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 94 11 9481

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A,D | TETRAHEDRON LETTERS., vol.34, no.28, 1993, OXFORD GB pages 4477 - 4480 D.F. HUANG ET AL. 'A versatile total synthesis of epibatidine and analogs' ----- | 1-16 | |
| | | | TECHNICAL FIELDS SEARCHED (Int.Cl.6) |

The present search report has been drawn up for all claims

| Place of search | Date of completion of the search | Examiner |
|---|---|---|
| THE HAGUE | 23 March 1995 | Bosma, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)